(19) Europäisches Patentamt
European Patent Office
Office européen des brevets

(11) EP 3 773 565 B1

(12) EUROPEAN PATENT SPECIFICATION

(45) Date of publication and mention of the grant of the patent:
**24.09.2025 Bulletin 2025/39**

(21) Application number: **19775315.5**

(22) Date of filing: **27.03.2019**

(51) International Patent Classification (IPC):
***A61K 31/4166*** *(2006.01)* ***A61P 35/00*** *(2006.01)*

(52) Cooperative Patent Classification (CPC):
**A61K 31/4166; A61P 35/00**

(86) International application number:
**PCT/CN2019/079893**

(87) International publication number:
**WO 2019/184952 (03.10.2019 Gazette 2019/40)**

# (54) DEUTERATED IMIDAZOLIDINEDIONE COMPOUNDS AND THEIR USES

DEUTERIERTE IMIDAZOLIDINDIONVERBINDUNGEN UND IHRE VERWENDUNG

COMPOSÉS D'IMIDAZOLIDINEDIONE DEUTÉRÉS ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2018 US 201862650153 P**

(43) Date of publication of application:
**17.02.2021 Bulletin 2021/07**

(73) Proprietor: **Hinova Pharmaceuticals Inc.**
**Chengdu, Sichuan 610041 (CN)**

(72) Inventors:
- **LI, Xinghai**
  **Chengdu, Sichuan 610041 (CN)**
- **CHEN, Yuanwei**
  **Chengdu, Sichuan 610041 (CN)**

(74) Representative: **Altmann Stößel Dick Patentanwälte PartG mbB**
**Theodor-Heuss-Anlage 2**
**68165 Mannheim (DE)**

(56) References cited:
**EP-A1- 3 795 153** **WO-A1-2013/087004**

- **BI F ET AL: "Phase I study of HC-1119, an androgen receptor inhibitor: Results from the dose escalation and expansion cohorts", JOURNAL OF CLINICAL ONCOLOGY 20180501 AMERICAN SOCIETY OF CLINICAL ONCOLOGY NLD, vol. 36, no. 15, Supplement 1, 1 May 2018 (2018-05-01), XP009531086, ISSN: 1527-7755**
- **XUEHAI PANG ET AL.: "Effect of N-methyl deuteration on pharmacokinetics and pharmacodynamics of enzalutamide", JOURNAL OF LABELLED COMPOUNDS AND RADIOPHARMACEUTICALS, vol. 60, no. 9, 31 December 2017 (2017-12-31), pages 401 - 409, XP055641210, ISSN: 0362-4803**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

Prostate cancer (prostatic carcinoma, abbreviated as PCa) is the most common malignant neoplasm in in male reproductive system. The incidence thereof increases with age, and differs significantly from region to region, which is higher in U.S. and Europe. Second to lung cancer, prostate cancer is the second cancer leading to death in men. Also, in the past, not much attention has been paid to prostate cancer in China, as it represented a small fraction of the diseases in the tumor spectrum. However, with the social development and progress in China, the aging of society, urbanization, westernization of dietary structure and advances in detection technology, the incidence of prostate cancer has significantly increased. A survey on prostate cancer which was completed by The Second Hospital of Tianjin Medical University and Diagnosis and Treatment of Prostate Cancer in Tianjin in 2011 showed that the incidence of prostate cancer in Tianjin was rapidly rising; the incidence of prostate cancer increased by 4 times in 20 years, and the number of patients with prostate cancer accounted for 13.4% of inpatient with urinary tract tumors. Prostate cancer which was a rare cancer in the past has become a common tumor in recent times, in many parts of the world.

An androgen receptor is a ligand-dependent trans-transcriptional regulatory protein with 110,000 dalton molecular weight. Androgen receptor plays a very important role in the pathogen and deterioration process of prostate cancer, and in male hormone-related diseases such as acne, male alopecia, and so on.

Traditional methods for treating prostate cancer include surgery or using androgen receptor antagonists such as bicalutamide (Casodex). However, patients tend to develop drug resistance after 2-4 years of treatment. Also, bicalutamide has side effects of stimulating the proliferation of cancer, and therefore patients must stop using bicalutamide after a period of time. Recent studies have found that bicalutamide will activate androgen receptors, thereby stimulating the proliferation of cancer.

Therefore, there is still a need in the art to develop compounds having superior pharmacodynamic properties for the treatment of prostate cancer.

Pang, Xuehai et. al report the synthesis of a deuterated analogue of Enzalutamide, a second-generation antiandrogen, which has been developed for the treatment of castration-resistance prostate cancer. The synthesized deuterated analogue showed higher drug exposure and thus stronger antitumor potency in preclinical settings (Pang, Xuehai, Lingling Peng, and Yuanwei Chen. "Effect of N-methyl deuteration on pharmacokinetics and pharmacodynamics of enzalutamide." Journal of Labelled Compounds and Radiopharmaceuticals 60.9 (2017): 401-409).

The present invention is defined by the appended claims. The references to methods of treatment by therapy of this description are to be interpreted as references to compounds, pharmaceutical compositions and medicaments of the present invention for use in those methods.

Provided herein is compound HC-1119, or a crystal form, pharmaceutically acceptable salt, hydrate or solvate thereof, for use in treating prostate cancer in a subject in need thereof, characterized in that said compound is administered to said subject in a therapeutically effective amount at a dosage of 80 mg/day, wherein the prostate cancer is castration resistant prostate cancer (CRPC) or metastatic castration resistant prostate cancer (mCRPC).

The present invention also relates to the compound HC-1119 for use in treating prostate cancer in a subject in need thereof, wherein upon administration of HC-1119 to the subject plasma concentrations of a combination of HC-1119 and its second metabolic product (HC-1119-M2) are produced that are at least 50% more than the plasma concentrations obtained with a combination of enzalutamide and its second metabolic product (M2) with an identical dosage of enzalutamide, wherein said compound is administered to said subject in a therapeutically effective amount at a dosage of 80 mg/day; wherein the prostate cancer is castration resistant prostate cancer (CRPC) or metastatic castration resistant prostate cancer (mCRPC). Further, the invention provides the compound HC-1119 for use in treating prostate cancer in a subject in need thereof, wherein upon administration of HC-1119 to the subject plasma concentrations of a combination of HC-1119 and its second metabolic product (HC-1119-M2) of at least about 10 μg/mL at steady state are produced, wherein the dosage of HC-1119 is 80 mg/day; wherein the prostate cancer is castration resistant prostate cancer (CRPC) or metastatic castration resistant prostate cancer (mCRPC). The invention also relates to the compound HC-1119 for use in treating prostate cancer in a subject in need thereof, wherein the inter-patient variability in the plasma concentrations or in the AUC is reduced compared to enzalutamide, characterized in that said compound HC-1119 is administered to a subject in need thereof in a therapeutically effective amount at a dosage of 80 mg/day; wherein the prostate cancer is CRPC or mCRPC.

Compound HC-1119 referred herein is an imidazolidinedione compound, 4-{3-[4-cyano-3-(trifluoromethyl) phenyl]-5,5-dimethyl-4-oxo-2-thio-1-imidazolidinyl}-2-fluoro-Ntrid euteromethyl benzamide of formula (I):

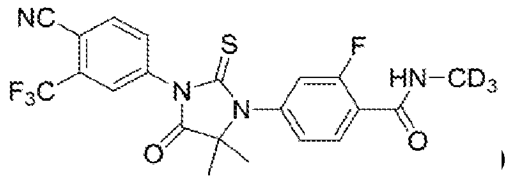

## BRIEF DESCRIPTION OF FIGURES

Figures 1A and 1B of Figure 1 show the results of the HC-1119 Phase 1a clinical studies.
Figures 2A and 2B show the results of the Phase I/II studies with the similar drug, enzalutamide.
Figure 3 provides the clinical PK parameters (40 mg QD) from the HC-1119 trial.
Figure 4 provides the clinical PK parameters (80 mg QD) from the HC-1119 trial.
Figure 5 provides the clinical PK parameters (160 mg QD) from the HC-1119 trial.
Figure 6 provides the clinical PK parameters (200 mg QD) from the HC-1119 trial.
Figure 7 provides the clinical PK parameters from an enzalutamide study.
Figure 8 provides some PK parameters for 80 mg HC-1119 in comparison with those of 160 mg of enzalutamide.
Figures 9 shows the plasma concentration versus time curve at week 12 for patients dosed with 40 mg HC-1119.
Figures 10 shows the plasma concentration versus time curve at week 12 for patients dosed with 80 mg HC-1119.
Figures 11 shows the plasma concentration versus time curve at week 12 for patients dosed with 160 mg HC-1119.
Figures 12 shows the plasma concentration versus time curve at week 12 for patients dosed with 200 mg HC-1119.
Figure 13 shows the M0 $C_{24h}$ changes during the 12 weeks of treatment for patients dosed with 40 mg, 80 mg, 160 mg and 200 mg of HC-1119.
Figure 14 shows the M1 $C_{24h}$ changes during the 12 weeks of treatment for patients dosed with 40 mg, 80 mg, 160 mg and 200 mg of HC-1119.
Figure 15 shows the M2 $C_{24h}$ changes during the 12 weeks of treatment for patients dosed with 40 mg, 80 mg, 160 mg and 200 mg of HC-1119.
Figures 16A and 16B show the (M0+M2) changes in $C_{24h}$ at steady state for patients dosed with either 80 mg HC-1119 (Figure 16A) or 160 mg enzalutamide (Figure 16B).

## DETAILED DESCRIPTION

The invention is based on the discovery that the deuterated imidazolidinedione compound of formula (I) of the present invention, or a crystal form, pharmaceutically acceptable salt, hydrate or solvate thereof have excellent pharmacokinetics and/or pharmacodynamic properties, and is, therefore, more suitably used as androgen receptor antagonists, and in the preparation of drugs/medicaments for treating androgen-related diseases. The invention provides the imidazolidinedione compound, 4-{3-[4-cyano-3-(trifluoromethyl)phenyl]-5,5-dimethyl-4-oxo-2-thio-1-imidazolidinyl }-2-fluoro-Ntrideuteromethyl benzamide, of formula (I) above, or a crystal form, pharmaceutically acceptable salt, hydrate or solvate thereof for use as defined by the claims.

The compound of the invention possesses outstanding androgen receptor (AR) antagonism, therefore, the compound of the invention and the crystal forms, the pharmaceutically acceptable inorganic or organic salts, hydrates or solvates thereof, and pharmaceutical compositions comprising the compound of the invention as the main active ingredient, can be used for treating, preventing and alleviating diseases mediated by the AR.

The terms "compound of the invention," the "compound of formula (I)" and "HC-1119" are used interchangeably herein and all refer to 4-{3-[4-cyano-3-(trifluoromethyl)phenyl]-5,5-dimethyl-4-oxo-2-thio-1-imidazolidinyl }-2-fluoro-N-trideuteromethyl benzamide.

A pharmaceutical composition of the invention comprises a safe and effective amount of the compound of the invention or the pharmaceutical acceptable salts thereof and pharmaceutically acceptable excipients or carriers. As used herein, a "safe and effective amount" refers to an amount of the compounds which is sufficient to improve the patient's condition and would not induce serious side effect.

As used herein, a "pharmaceutically acceptable carrier" refers to one or more compatible solid or liquid fillers or gel material, which are suitable for human, and must have sufficient purity and sufficiently low toxicity. "Compatibility" herein means that the components of the compositions can be blended with the compounds of the invention or with each other, and would not significantly reduce the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate,

vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc.

In one aspect, the invention provides compound HC-1119, or a crystal form, pharmaceutically acceptable salt, hydrate or solvate thereof, for use in treating prostate cancer in a subject in need thereof, characterized in that said compound is administered to said subject in a therapeutically effective amount at a dosage of 80 mg/day, wherein the prostate cancer is castration resistant prostate cancer (CRPC) or metastatic castration resistant prostate cancer (mCRPC).

According to an embodiment, the treatment reduces the plasma concentration of prostate specific antigen (PSA) in a subject with elevated PSA.

In one embodiment, the plasma concentration of PSA in a subject with elevated PSA is lowered by at least 50%. In another embodiment, the plasma concentration of PSA in a subject with elevated PSA is lowered by at least 55%. In another embodiment, the plasma concentration of PSA in a subject with elevated PSA is lowered by at least 60%. In another embodiment, the plasma concentration of PSA in a subject with elevated PSA is lowered by at least 65%. In another embodiment, the plasma concentration of PSA in a subject with elevated PSA is lowered by at least 70%. In another embodiment, the plasma concentration of PSA in a subject with elevated PSA is lowered by at least 75%. In another embodiment, the plasma concentration of PSA in a subject with elevated PSA is lowered by at least 80%. In another embodiment, the plasma concentration of PSA in a subject with elevated PSA is lowered by at least 85%.

In another embodiment, the plasma concentration of PSA in a subject with elevated PSA is lowered by over 89%. In yet another embodiment, the plasma concentration of PSA in a subject with elevated PSA is lowered by at least 90%. In some embodiments, the plasma concentration of PSA in a subject with elevated PSA is lowered by at least 91% or at least 92% or at least 93% or at least 94%. In some embodiments, the plasma concentration of PSA in a subject with elevated PSA is lowered by at least 95% or at least 96% or at least 97% or at least 97.5%. In some embodiments, the plasma concentration of PSA in a subject with elevated PSA is lowered by at least 98% or at least 98.5% or at least 99% or at least 99.5%. In some embodiments, the plasma concentration of PSA in a subject with elevated PSA is lowered by at least 99.6% or at least 99.7% or at least 99.8% or at least 99.9%.

In one embodiment, the plasma concentration of PSA in a subject with elevated PSA is lowered by at least 50% over a period of five (5) weeks or more. In another embodiment, the plasma concentration of PSA in a subject with elevated PSA is lowered by at least 50% over a period of six (6) weeks or more. In yet another embodiment, the plasma concentration of PSA in a subject with elevated PSA is lowered by at least 50% over a period of eight (8) weeks or more. In yet another embodiment, the plasma concentration of PSA in a subject with elevated PSA is lowered by at least 50% over a period of ten (10) weeks or more. In yet another embodiment, the plasma concentration of PSA in a subject with elevated PSA is lowered by at least 50% over a period of twelve (12) weeks or more.

Prostate-specific antigen (PSA) is a protein produced by the prostate gland. Elevated PSA levels may indicate prostate cancer or a noncancerous condition such as prostatitis, or an enlarged prostate. Most men have PSA levels under 4 ng/mL and this has traditionally been used as the cutoff for concern about the risk of prostate cancer.

For this reason, measurement of PSA in the blood has been used as a screening test for prostate cancer. However, the PSA test was first developed only to monitor men who had a history of prostate cancer. No single normal level has been established. Historically, a level of 4.0 ng/mL or higher was used to justify a biopsy of the prostate (a sample of prostate tissue) to try and determine if a man has prostate cancer. However, this practice has been changing and other factors are being considered in the decision to perform a prostate biopsy.

As used herein, a subject with an "elevated PSA concentration" or "elevated PSA level" has a PSA concentration that is about 5 ng/ml or higher. In some embodiments, The PSA concentration in a subject with an elevated PSA concentration is about 6 ng/ml or higher or about 7 ng/ml or higher or about 8 ng/ml or higher or about 9 ng/ml or higher. In one embodiment, the PSA concentration in a subject with an elevated PSA concentration is about 10 ng/ml or higher.

The terms "subject" or "patient" are used interchangeably herein to mean all mammals including humans. Examples of subjects include humans, cows, dogs, cats, horses, goats, sheep, pigs, and rabbits. In one embodiment, the patient is a human.

In one embodiment, the subject has previously been diagnosed with prostate cancer. Said prostate cancer could be any type of prostate cancers. The prostate cancer could be any type of AR positive prostate cancers, including, but not limited to, castration resistant prostate cancer (CRPC) or metastatic castration resistant prostate cancer (mCRPC) or non-metastatic castration resistant prostate cancer (nmCRPC).

The subject could be one who is new to any form of treatment for prostate cancer, or one who has previously been treated for prostate cancer. In one embodiment, the subject is one who has never been treated for prostate cancer. In another embodiment, the subject was previously diagnosed with prostate cancer and has undergone previous treatment for prostate cancer.

In one embodiment, the treatment reduces the plasma concentration of prostate specific antigen (PSA) in the subject with elevated PSA by at least 50% comprising administering to said subject at least 80 mg/day of HC-1119.

The compound of this invention, or a crystal form, pharmaceutically acceptable salt, hydrate or solvate thereof or a pharmaceutical composition comprising HC-1119, may be administered by any number of routes including, but not limited to, oral, parenteral, intravenous, intramuscular, intra-arterial/intraarticular, intramedullary, intraperitoneal, intrathecal, intraventricular, transdermal, transcutaneous, topical, subcutaneous, intranasal, transmucosal, enteral, dermal, buccal, sublingual, intraocular, intravaginal or rectal routes. Hyposprays may also be used to administer the pharmaceutical compositions of the invention. Typically, the therapeutic composition may be prepared as injectables, either as liquid solutions or suspensions. Solid forms suitable for solution in, or suspension in, liquid vehicles prior to injection may also be prepared. In one embodiment, the therapeutic compound or composition is administered orally. In another embodiment, the therapeutic compound or composition is administered as an injection, or as a capsule, tablet, pill, powder or as granules.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and gum arabic; (c) humectant, such as, glycerol; (d) disintegrating agents such as agar, calcium carbonate, potato starch or tapioca starch, alginic acid, certain complex silicates, and sodium carbonate; (e) dissolution-retarding agents, such as paraffin; (f) absorption accelerators, for example, quaternary ammonium compounds; (g) wetting agents, such as cetyl alcohol and single glyceryl stearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage forms may also contain buffer.

The solid dosage forms such as tablets, sugar pills, capsules, pills and granules can be prepared by using coating and shell material, such as enteric coatings and other materials known in the art. They can contain opaque agent, and the release of the active compounds or compounds in such compositions can be delayed for releasing in certain portion of the digestive tract. Instance of the embedding components can be polymers and waxes. If necessary, the active compounds and one or more above excipients can be prepared into microcapsules.

Liquid dosage forms for oral administration include pharmaceutically acceptable emulsions, solutions, suspensions, syrups or tinctures. In addition to the active compounds, the liquid dosage forms may contain conventional inert diluent known in the art, such as water or other solvent, solubilizer and emulsifier, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the mixtures thereof and so on.

Besides the inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or the mixtures thereof and so on.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and the suitable mixtures thereof.

The dosage forms of the compound of the invention for topical administration include ointments, powders, patches, aerosol, and inhalants. The active ingredients are mixed with physiologically acceptable carriers and any preservatives, buffers, or propellant if necessary, under sterile conditions.

The compound of the invention can be administered alone, or in combination with other pharmaceutically acceptable compounds.

In another aspect, the invention provides a method of treating a male hormone-related disease or condition in a subject with elevated PSA comprising administering HC-1119, or a crystal form, pharmaceutically acceptable salt, hydrate or solvate thereof or a pharmaceutical composition comprising HC-1119 to the subject.

As described above, the male hormone-related disease is prostate cancer wherein said cancer is castration resistant prostate cancer (CRPC) or metastatic castration resistant prostate cancer (mCRPC). The subject could be one who has been previously diagnosed or is currently diagnosed with the disease or condition. The subject may, but need not, have been previously treated for the disease or condition.

In another aspect, the treatment of the invention produces plasma concentrations of a combination of HC-1119 and its second metabolic product (HC-1119-M2) in a subject that is at least 50% more than a combination of the enzalutamide and its second metabolic product (M2) produced with an identical dosage of enzalutamide.

The structural formula for HC-1119, 4-{3-[4-cyano-3-(trifluoromethyl)phenyl]-5,5-dimethyl-4-oxo-2-thio-1-imidazolidinyl}-2-fluoro-N-trideuteromethyl benzamide, is provided above as formula (I). As would be known to one of skill in the art, enzalutamide has the same structural formula except that in the case of enzalutamide the hydrogens on the amide methyl ($-CH_3$) group remain hydrogens and are not substituted with deuterium. These compounds, enzalutamide and

HC-1119 are also referred to herein as M0 and HC-1119-MO, respectively.

Provided below are the first metabolic product (M1) and second metabolic product (M2), respectively, of enzalutamide and HC-1119. Based on *in vitro* experiments, M1 is not active, whereas M2 exhibited similar *in vitro* activity to enzalutamide. While it is noted (based on the structures below) that deuteration (at the now eliminated methyl group) is irrelevant in the case of M1 and M2, just in order to distinguish the source of the metabolic product, the first and second metabolic product of HC-1119 will be referred to as HC-1119-M1 and HC-1119-M2, respectively, whereas the first and second metabolic product of enzalutamide will be referred to as M1 and M2, respectively. M1 is identical to HC-1119-M1 and M2 is identical to HC-1119-M2.

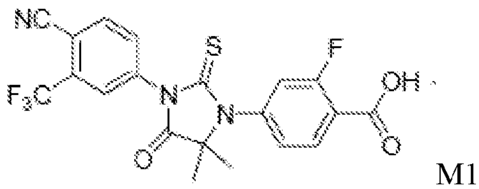

M1 (or HC-1119-M1)

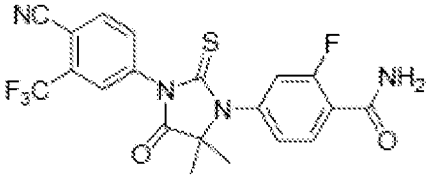

M2 (or HC-1119-M2)

In one embodiment, the treatment of the invention produces plasma levels of the combination of HC-1119-M0 (HC-1119) and HC-1119-M2 (the second metabolic product of HC-1119) of about 10 μg/ml at steady state. In another embodiment, the plasma levels of the combination of HC-1119-M0 and HC-1119-M2 are >10 μg/ml. In yet another embodiment, the plasma levels of the combination of HC-1119-MO and HC-1119-M2 are about 20 μg/ml. In yet another embodiment, the plasma levels of the combination of HC-1119-MO and HC-1119-M2 are about 30 μg/ml. In yet another embodiment, the plasma levels of the combination of HC-1119-MO and HC-1119-M2 are about 40 μg/ml. In yet another embodiment, the plasma levels of the combination of HC-1119-MO and HC-1119-M2 are about 50 μg/ml. In yet another embodiment, the plasma levels of the combination of HC-1119-MO and HC-1119-M2 are >50 μg/ml.

The above mentioned plasma levels of the combination of HC-1119-MO and HC-1119-M2 can be achieved by administrating to the subject 80 mg/day of HC-1119.

In one embodiment, the invention provides a method of producing plasma concentrations of a combination of HC-1119 and HC-1119-M2 in a subject that is at least about 10 μg/mL at steady state by administering 80 mg/day HC-1119.

In another embodiment, the treatment of the invention produces plasma concentrations of a combination of HC-1119 and HC-1119-M2 in a subject that is at least about 20 μg/mL at steady state by administering 80 mg/day HC-1119.

In yet another aspect, the treatment of the invention reduces inter-patient variability in the plasma concentrations and/or AUC in comparison to enzalutamide. The reduced inter-patient variability may, in one embodiment, be demonstrated by, for example, a smaller percent coefficient of variation (CV%)of the standard deviation (SD) comparing to that of enzalutamide. In one embodiment, the inter-patient variability in $C_{max}$, $C_{24h}$, and/or $C_{trough}$ are reduced.

A nonsteroidal antiandrogen (NSAA) compound is an antiandrogen compound with a nonsteroidal chemical structure. They are typically selective and full or silent antagonists of the androgen receptor (AR) and act by directly blocking the effects of androgens like testosterone and dihydrotestosterone. NSAAs are used in the treatment of androgen-dependent conditions in men and women. Unlike steroidal antiandrogen compounds, NSAAs have little or no capacity to activate the AR, show no off-target hormonal activity such as progestogenic, glucocorticoid, or antimineralocorticoid activity, and lack antigonadotropic effects. For these reasons, they have improved efficacy and selectivity as antiandrogens and do not lower androgen levels. Instead, they act solely by directly blocking the actions of androgens at the level of their biological target, the AR.

Inter-patient variability in the plasma concentrations(for example, $C_{max}$, $C_{24h}$, or $C_{trough}$) or AUC of in comparison to enzalutamide can be reduced by administering to a patient in need thereof, a 80 mg/day HC-1119.

As described above, the subject or patient has The subject could be one who has been previously diagnosed or is currently diagnosed with the disease or condition. The subject may, but need not, have been previously treated for the disease or condition.

In one embodiment, the subject is one who was previously diagnosed with prostate cancer and has undergone previous treatment for prostate cancer. The subject could be who was diagnosed with any type of prostate cancer,

including, but not limited to, CRPC and mCRPC and nmCRPC, and has undergone previous treatment for the same. In another embodiment, the subject is one who has never been treated for prostate cancer.

In the context of the present invention, the NSAA compound is enzalutamide and the deuterated analogue of the NSAA compound is HC-1119.

In one embodiment, HC-1119 is administered over a period of at least five (5) weeks. In some embodiments, HC-1119 is administered over a period of at least six (6) weeks or at least eight (8) weeks, or at least ten (10) weeks, or at least twelve (12) weeks.

**General**

As used herein, the term "deuterated" means that hydrogen(s) in a compound or group is substituted by deuterium(s). "Deuterated" can be mono-substituted, bi-substituted, multi-substituted or total-substituted. The terms "one or more deuterium-substituted" and "substituted by deuterium for one or more times" can be used interchangeably.

In one embodiment, the deuterium content in a deuterium-substituted position is greater than the natural abundance of deuterium (0.015%). In some embodiments, the deuterium content in a deuterium-substituted position is greater than 50%, or greater than 75%. In some embodiments, the deuterium content in a deuterium-substituted position is greater than 90%, or greater than 95%, or greater than 97%, or greater than 99%, or greater than 99.5%.

As used herein, the term "compound of the invention" refers to the compound of formula (I). This term also includes various crystal forms, pharmaceutically acceptable salts, hydrates or solvates of the compound of formula (I). As stated above, these terms are used interchangeably herein with "HC-1119" and all refer to 4-{3-[4-cyano-3-(trifluoromethyl)phenyl]-5,5-dimethyl-4-oxo-2-thio-1-imidazolidinyl }-2-fluoro-N-trideuteromethyl benzamide.

As used herein, the term "pharmaceutically acceptable salt" refers to the salts which are suitable for medicine and formed by the compound of the invention with an acid or a base. Pharmaceutically acceptable salts include inorganic salts and organic salts. A preferred salt is formed by the compound of the invention with an acid. The acid suitable for forming salts includes, but not limited to, inorganic acid, such as hydrochloric acid, hydrobromic acid, hydrofluoric acid, sulfuric acid, nitric acid, phosphoric acid; organic acid, such as formic acid, acetic acid, propionic acid, oxalic acid, malonic acid, succinic acid, fumaric acid, maleic acid, lactic acid, malic acid, tartaric acid, citric acid, picric acid, methanesulfonic acid, benzene methanesulfonic acid, benzene sulfonic acid; and acidic amino acid, such as aspartic acid, glutamic acid.

As used herein, the terms "study" and "trial" are used interchangeably to refer to a clinical trial.

As used herein, the terms "Stage" and "Phase" are used interchangeably to refer to the different stages of a clinical trial.

The term "comprising" encompasses "including" as well as "consisting" *e.g.,* a composition "comprising" X may consist exclusively of X or may include something additional *e.g.,* X + Y.

The term "about" in relation to a numerical value x means x $\pm$ 10%.

As used herein, reference to "treatment" of a subject or patient is intended to include prevention, prophylaxis, attenuation, amelioration, alleviation and therapy.

The term "disease" as used herein is intended to be generally synonymous, and is used interchangeably with, the terms "disorder" and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

**EXAMPLES**

Examples of embodiments of the present invention are provided in the following examples. The following examples are presented only by way of illustration and to assist one of ordinary skill in using the invention. The examples are not intended in any way to otherwise limit the scope of the invention.

**Example 1. Preparation of the compound of the invention**

The preparation of HC-1119 of the present invention is described in detail below. However, these specific methods are not provided for the limitation of the invention. The compound of the invention can be readily prepared by optionally combining any of the various methods described in the specification or various methods known in the art, and such combination can readily be carried out by one skilled in the art.

In general, during the preparation, each reaction is conducted in solvent, at a temperature between room temperature to reflux temperature (such as 0-120°C, preferably 0-80°C.). Generally, the reaction time is 0.1-60 hours, preferably, 0.5-48 hours.

In one embodiment, the preparation method for HC-1119, 4-{3-[4-cyano-3-(trifluoromethyl)phenyl]-5,5-dimethyl-4-oxo-2-thio-1-imidazolidinyl }-2-fluoro-N-trideuteromethylbenzamide is as follows:

Synthesis of 2-fluoro-N-trideuteromethyl-4-nitro-benzamide (compound B):

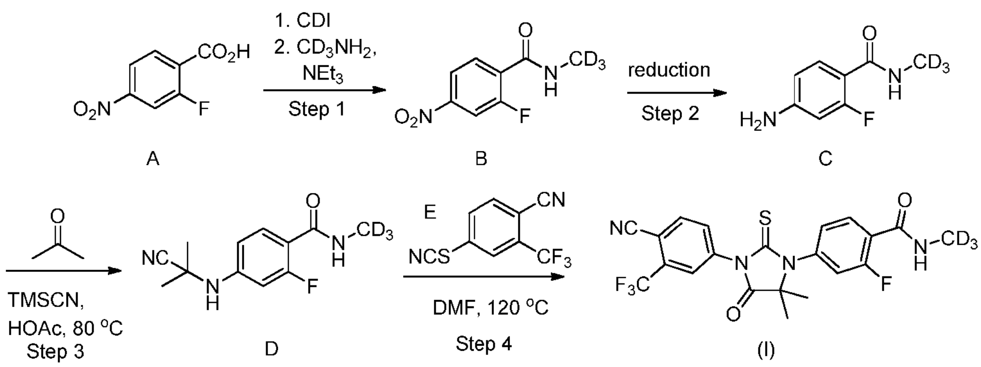


Into a solution of compound A (5.25 g, 28.37 mol) in dichloromethane (20 mL) CDI (4.62 g, 28.37 mmol) was added. The reaction mixture was stirred at room temperature for one hour. Into a solution of trideuterated methylamine hydrochloride (2 g, 28.76 mmol) in methylene chloride (20 mL) triethylamine (3.27 g, 32.36 mmol) was added to give a white suspension. After stirred at room temperature for half an hour, the suspension was added to the reaction mixture slowly. The resulting mixture was stirred for another hour, the reaction was quenched by adding water (10 mL). The organic phase was separated and the aqueous phase was extracted with dichloromethane (2 x 20 mL) twice. The organic phases were combined, washed with 1 M hydrochloric acid (2 x 10 mL) twice, 1 M aqueous sodium hydroxide solution (2 x 10mL) twice and saturated brine (10 mL) once, dried ($Na_2SO_4$), filtered and concentrated under reduced pressure to give a white solid compound B (compound B, 5.1 g, 88.2% yield); MS: 202(M + $H^+$).

Synthesis of 4-amino-2-fluoro-*N*-trideuteromethyl benzamide (Compound C):

Compound B (5.1 g, 25.37 mmol) was dissolved in EtOAc and acetic acid (15 mL + 5 mL) to form a solution. Then to this solution was added iron powder (15 g) and the reaction refluxed overnight for 16 h. The reaction was cooled to room temperature and precipitate was filtered and washed with EtOAc 3 times (3 x 20 mL). The organic solutions were combined, washed with brine, dried over sodium sulfate, concentrated to give yellow solid. It was further purified by flash chromatography (DCM:MeOH = 50:1) to give compound C as a pale yellow solid (2.22 g, 51.2% yield). $^1H$ NMR ($CDCl_3$, 400 MHz): $\delta$ (ppm) 7.92 (1H, t, J = 8.8 Hz), 6.59 (1H, s), 6.49 (1H, d, J = 8.4 Hz), 6.32(1H, d, J = 14.4 Hz), 4.10 (2H,s).

Synthesis of 4-(2-cyano-2-propylamino)-2-fluoro-N-trideuteromethyl-benzamide (compound D):

TMSCN (4 g, 40.3 mmol) and compound C (1.5 g, 8.76 mmol) were dissolved in a mixed solution of acetic acid (10 mL) and acetone (10 mL). The resulting mixture was maintained in a sealed tube at 80°C overnight (16 h), and then cooled to room temperature. Acetone was removed under reduced pressure, water was added (20 mL), and the resulting mixture was extracted with ethyl acetate, washed with brine, dried over sodium sulfate, and concentrated. The resulting solid was washed with petroleum ether (10 mL) and dried by suction to give compound D as a white solid (1.95 g, 93.4% yield).

Synthesis of 4-{3-[4-cyano-3-(trifluoromethyl)phenyl]-5,5-dimethyl-4-oxo-2-thio-1 -imidazolidinyl}-2-fluoro-N-trideuteromethyl benzamide (HC-1119):

Compound D (0.5 g, 2.1 mmol) and compound E (0.5 g, 2.19 mmol) were dissolved in DMF (10 mL).The resulting mixture was heated to 120°C overnight (16 h). Ethanol (5 mL), water (5 mL) and concentrated hydrochloric acid (1 mL) were added, and the resulting mixture was heated at reflux for 1 h. The mixture was extracted with ethyl acetate, washed with brine, dried over sodium sulfate, and concentrated. The residue was purified by column chromatography (PE: EA / 1: 1) to give a brown solid which was further purified by preparative chromatography to give compound (I), HC-1119 as a brown solid (132.7mg, 11% yield). $^1H$ NMR (DMSO-$d_6$, 400MHz): $\delta$ (ppm) 8.44 (1H, s), 8.41 (1H, d, J = 8.4Hz), 8.30 (1H, s), 8.09 (1H, d, J = 7.6Hz), 7.79 (1H, t, J = 8Hz), 7.44 (1H, d, J = 11.2Hz), 7.34 (1H, d, J = 8.8Hz), 1.54 (6H, s). MS: 477.2 (M + $H^+$).

**Example 2: Description of open-label clinical trial to evaluate tolerability, pharmacokinetics and efficacy in patients with metastatic castration-resistant prostate cancer**

An open-label study of soft gel capsules of HC-1119 was conducted on patients with metastatic castration-resistant prostate cancer (mCRPC). There were two (2) stages or arms to the study, Stage A and Stage B.

Stage A had four (4) dose-escalation cohorts of 40 mg, 80 mg, 160 mg, and 200 mg of HC-1119.During Stage A, the dose escalation tolerance (or tolerability) study was conducted using the traditional rule-based 3 +3 design. All subjects in Stage A were involved in pharmacokinetic studies. Cohorts 80 mg and 160 mg were each increased to 8 subjects to participate in the PK study. Table 1 provides a summary of Stage A.

**Table 1.**

| Cohort | Drug | Dose | Dose Escalation | Increased cases for PK study | Total |
|---|---|---|---|---|---|
| 1 | HC-1119 | 40 mg | 3 | 0 | 3 |
| 2 | HC-1119 | 80 mg | 3 | 5 | 8 |
| 3 | HC-1119 | 160 mg | 3 | 5 | 8 |
| 4 | HC-1119 | 200 mg | 3 | 0 | 3 |
| 5* | HC-1119 | 120 mg | 3 | 5 | 8 |
| * If Dose Limiting Toxicity (DLT) occurred in ≥2/6 subjects in the 160 mg dose group, the dose will be reduced to 120 mg for tolerance and pharmacokinetic studies. | | | | | |

**Example 3. Study Objectives**

The primary outcome measures were to evaluate the tolerability and safety of HC-1119 in patients with mCRPC and to explore the dose-limiting toxicity (DLT).

The secondary outcome measures were to assess the pharmacokinetic (PK) parameters of HC-1119 and to assess the pharmacodynamics characteristics of HC-1119 on serum PSA.

**Example 4. Dose-escalation Regimen**

Starting dose estimation: The similar drug enzalutamide had good safety results in the dose range of 30 mg - 240 mg, with the maximum human exposure being 600 mg and DLT occurring at the dose of 360 mg. With enzalutamide, the initial dose in the human trials was 30 mg of enzalutamide.

Preclinical studies have shown that, under similar exposures, the toxicological characteristics and the target organ of HC-1119 are similar to those of enzalutamide.

The highest non-severely toxic dose (HNSTD) for beagle dogs in repeated dose toxicity tests (28 days) was 90 mg/kg. In this conversion, adult equivalent dose (HED) was calculated to be: 0.54 * 90mg/kg * 60 kg = 2916 mg/d. General antineoplastic drugs in the human body are safe at an initial dose of 1/6 HNSTD. This set the initiator at 486 mg/day. This was then divided by an additional safety factor of 12, setting the starting dose at set at 40 mg/day of HC-1119.

The maximum tolerable dosage (MTD) in the Phase I dose escalation study of the similar drug, enzalutamide, was 240 mg/d. A fifth (1/5) of that provides a dose of 48 mg/d.

The clinical treatment dose during the study with the similar drug, enzalutamide, was 160mg/d. Pre-clinical pharmacodynamics studies showed that the activity of HC-1119 was better than that of enzalutamide. Choosing the initial dose of 40 mg/d was safe and reduced the risk of exposure to ineffective doses. After comprehensive safety and ethical considerations, 40 mg/d was chosen to be the initial dose of the dose-escalation test.

The maximum dose: Preclinical efficacy studies showed that HC-1119 activity was superior to the similar drug, enzalutamide. The maximum tolerated dose of enzalutamide was 240 mg/d and its clinical dosage was 160mg/d. Therefore, the maximum dose of this study was designed to 200 mg/d.

Based on the test results, if more than 200 mg needed to be further explored, the researchers and the sponsor were to discuss whether to continue the dose escalation tolerance study in the higher dose group.

Dose-escalation Rules: The following were the dose-escalation rules that were followed during this study/trial.

(1) After the initial dose, according to the modified Fibonacci method, the dose was carried out from low to high, which were respectively 80 mg, 160 mg and 200 mg dose groups, and the subjects were enrolled in the traditional 3 + 3 mode.
(2) Each dose group first enroll three subjects. Every subject was observed after a single administration of HC-1119 for

24 hours. If no DLT appeared, then the subject entered the continuous administration period. If no DLT occurred in the 3 enrolled subjects, additional subjects were enrolled to a higher dose group. If DLT was present in 1 of the 3 enrolled subjects, then 3 subjects were re-enrolled in this dose group, and if no DLT was seen in the 3 re-enrolled subjects, then subjects were enrolled in the next higher dose group.

(3) If there were greater than 2/6 subjects with DLT in the 160 mg dose group, the dose was reduced to 120 mg for the tolerability study.

**Example 5. Dose-limiting Toxicity (DLT)**

Definition: Adverse events are those that occur during the dosing period (within 42 days) that may be associated with HC-1119 and meet any of the following criteria according to the NCI-CTC AE 4.03 grading criteria, will be considered DLT:

(1) Grade III / IV epilepsy, spasticity, or Grade II epilepsy (brief generalized seizures) in which case administration of HC-1119 was stopped, based on the judgment of the investigators;
(2) Hematological toxicity: Degree IV drug-related hematology AEs; Degree III neutropenia accompanied by significant fever (higher than 38.5 °C);
(3) Non-hematological toxicity: Degree III/IV drug-related AEs;
(4) Hair loss, Degree III/IV fever, nausea, vomiting, fatigue that were not accompanied with neutropenia were not considered as DLT; and
(5) Any level of toxicity that required termination of treatment, based on the judgment of the investigator and the sponsor.

**Example 6. Preliminary Efficacy Evaluation**

Stage A: PSA was dynamically observed to assess the initial dose-effectiveness based on data from subjects in each dose group until the end of the 12-week trial period. Serum PSA test, safety evaluation (including blood, liver and kidney function, electrocardiogram, etc.) were performed at the 6th, 8th, 10th and 12th weeks of visit, and the trial ended at the 12th week of visit.

Stage B: A dynamic PSA study was conducted to assess the initial dose-effectiveness of each dose group based on data from the subject's use of the test drug until the 12-week 'weekend. Serum PSA testing was performed at Weeks 3, 6, and 12 and ended at Week 12 of visit.

**Example 7. Pharmacokinetic studies**

All subjects in Stage A were involved in pharmacokinetic studies, and for the Dose Group of 80 mg and 160 mg each group was increased to 8 subjects to involve in the PK study. If there were more than 2/6 subjects with DLT in the 160 mg dose group, the dose was reduced to 120 mg for tolerability studies. If the 120 mg dose was tolerable, then the number of subjects was increased to 8 cases for PK study.

Single dose biological sample collection: Subjects were given a single fasting dose on the first day of the study. Blood samples were collected before ( -0.5~0h ) and after administration 0.5, 0.75, 1, 2h (±5min), 4, 8, 12 h (±15min), 24h (±30min, Day 2 before administration).Venous blood (3 mL) was collected, set in an anticoagulant tube (type of anticoagulant was based on established analytical test methods).

Continuous administration of biological samples collected: Blood sample collected on continuous administration Day 7, 21, 35, 42, 56, 70, 84 before administration, on Day 84 after administration 0.5, 0.75, 1, 2h (±5min), 4, 8, 12 h (±15min), 24h (±30min, Day 85 before administration). Venous blood (3 mL) was collected, set in an anticoagulant tube (type of anticoagulant was based on established analytical test methods).

According to the observed PK data, determined by the researcher and the sponsor, the specific time, frequency and quantity of PK sample collection was adjusted.

Sample Handling: Samples were centrifuged (about 3000 rpm for 10 minutes) to separate the plasma as soon as possible. Isolated plasma samples were stored in duplicate in plasma cryostat, with the tagged population's enrollment number, subject's initials, date and time of collection. Plasma samples were stored in -60 °C ~ -80 °C freezer as soon as possible. One blood sample was retained at the research center and one blood sample was shipped to a specialist testing company for plasma drug concentration determination. Compounds tested included HC-1119 prototype (M0) and metabolites M1, M2.

**Example 8. Eligibility Criteria**

Eligible subjects needed to have met/provided all of the following criteria:

(1) Willing and able to provide informed consent;
(2) Men, 18 years of age or older;
(3) Histologically or cytologically confirmed adenocarcinoma of the prostate, without neuroendocrine carcinoma or ductal adenocarcinoma;
(4) Evidence of distant metastatic disease (such as bone scans and CT / MRI findings);
(5) Castration (surgery or drugs), or combination with androgen deprivation therapy were relapsed, invalid, or advanced (disease progression was defined as one or more of the following three episodes occurred in the subject: (a) PSA progression, defined as a continuous increase in PSA levels of at least 3 measurements (≧ 1 week interval), with a> 50% increase from the lowest and PSA levels> 2 ng / mL at enrollment; (b) Progression of soft tissue disease as defined by RECIST 1.1; (c) Bone disease progression as defined by PCWG2, two or more new lesions found on bone scans);
(6) Total testosterone < 50ng/dl;
(7) Subjects who had not undergone bilateral orchidectomy must maintain effective GnRH analogue treatment throughout the study;
(8) Estimated survival time > 6 months;
(9) ECOG performance status ≤ 1;
(10) Laboratory tests meet the following criteria: (a) Blood tests: hemoglobin (Hb) ≥ 90 g/L (no blood transfusion within 14 days); Absolute Neutrophil Count (ANC) ≥ 1.5 × $10^9$/L; PLT ≥ 80 × $10^9$/L; (b) Blood biochemical tests: Serum creatinine (Cr) ≤ 2 × upper limit of normal (ULN), or creatinine clearance (CrCl) calculated ≥ 60 mL/min when serum creatinine> 2 × upper limit of normal (ULN) in subjects. Bilirubin BIL ≤ 2 × ULN. Alanine aminotransferase (ALT), aspartate aminotransferase (AST) ≤ 2.5 × ULN (subjects with liver metastases ≤ 5 × ULN); and (c) Coagulation function: International normalized ratio (INR) < 1.5.

**Example 9. Subject Exclusion Criteria**

Subjects were ineligible for inclusion, or were excluded from the study if they met any one of the following criteria:

1) Did not recover after a toxicity of Grade 2 or above after original treatment;
2) Clinically significant gastrointestinal abnormalities that could affect drug intake, transport or absorption (e.g., inability to swallow, chronic diarrhea, intestinal blockage, etc.) or total gastrectomy;
3) Allergies, or known to have a history of allergy to the drug components;
4) Metastases in the brain;
5) History of another malignancy within the previous 5 years (except for non-melanoma skin cancers that had been cured);
6) History of organ transplant;
7) HIV antibody positive;
8) Previous history of epilepsy or severe central nervous system disease history;
9) Unexplained coma history;
10) Family history of epilepsy;
11) History of traumatic brain injury;
12) History of drug abuse;
13) Serious cardiovascular disease, myocardial infarction, or arterial thrombosis, or unstable angina in the past 6 months, or in patients with clinical symptoms of heart failure;
14) Poorly controlled hypertension (systolic blood pressure ≥ 160 mmHg or diastolic blood pressure ≥ 100 mmHg), patients with a history of hypertension were allowed to participate in the study if their blood pressure was controlled by antihypertensive therapy;
15) Must combine with the use of drugs known to reduce the threshold of seizures during the trial;
16) Received 5α-reductase inhibitors (Finasteride, Dutasteride), Estrogen or Cyproterone treatment within 4 weeks;
17) Received ketoconazole treatment within 4 weeks;
18) Previously used investigational drugs or listed drugs that blocked androgen synthesis (such as Abiraterone Acetate, TAK-683, TAK-448) or against androgen receptors (such as enzalutamide, SHR3680, GT0918 (Proxalutamide), ARN509);
19) Participated in other drug clinical trials within 1 month prior to enrollment; or
20) Unsuitable to participate in this study as determined by the researchers.

**Example 10. Results of HC-1119 Phase Ia Studies**

Figures 1A and 1B of Figure 1 show the results of the HC-1119 Phase 1a clinical studies. They show the percentage change in PSA levels for subjects in the study. In both figures, the vertical axis represents the percent change in PSA levels from the baseline and the horizontal axis (numbers that are in line with the "0%") identifies individual patient or subject numbers.

Figure 1A shows the response rate at week 12 of the study. The rates of descent or decrease in PSA levels in the dose groups 40 mg, 80 mg, 160 mg and 200 mg by more than 50% from the baseline were 2/3 (66.7%), 6/8 (75%), 4/7 (57.1%), and 1/3 (33.3%), respectively. Two (2) patients, one in the dose group 80 mg and another in the dose group 160 mg, did not complete the trial.

Figure 1B shows the maximum response rate for subjects in the study. The maximum descent or decrease in PSA levels in the dose groups 40 mg, 80 mg, 160 mg and 200 mg by more than 50% from the baseline were 2/3(66.7%), 8/9 (88.8%), 4/8 (50%) and 3/3 (100%), respectively.

Figures 2A and 2B show the results of the Phase I/II studies with the similar drug, enzalutamide. Figures 2A and 2B show the percentage change in PSA levels for subjects in the study. In both figures, the vertical axis represents the percent change in PSA levels from the baseline and the horizontal axis identifies individual patient numbers. The solid lines show the decrease in PSA levels at 12 weeks for patients who stayed on treatment for at least 12 weeks; the dashed lines show decrease at less than 12 weeks for patients who did not stay on treatment for at least 12 weeks.

Figure 2A shows the results for subjects dosed with 60 mg/day (27 subjects), 150 mg/day (28 subjects), and 240 mg/day (29 subjects). Figure 2B shows the results for subjects dosed with 360 mg/day (28 subjects), 480 mg/day (22 subjects), and 600 mg/day (3 subjects).

**Example 11. Inter-patient Variability is reduced with HC-1119**

As provided above, inter-patient variability in the plasma concentrations and/or AUC of subjects administered with HC-1119 is reduced in comparison with those that receive the non-deuterated drug, enzalutamide.

Figure 3 provides the clinical PK parameters (40 mg QD) from the HC-1119 trial.

Figure 4 provides the clinical PK parameters (80 mg QD) from the HC-1119 trial.

Figure 5 provides the clinical PK parameters (160 mg QD) from the HC-1119 trial.

Figure 6 provides the clinical PK parameters (200 mg QD) from the HC-1119 trial.

Figure 7 provides the clinical PK parameters from an enzalutamide study. The data was obtained from FDA NDA documents and study number 9785-CL-0007.

The extent of inter-patient variation or variability was measured by the coefficient of variation (CV%). It can be seen from the tables provided in Figures 3-7 that HC-1119 demonstrated reduced inter-patient variation compared to enzalutamide after reaching steady state. Figure 7 shows steady state clinical PK parameters of enzalutamide (160 mg QD for 49 days). For M0, the CV for $C_{max}$, $C_{min}$, and $AUC_{tau}$ are 23.0%, 29.3% and 26.6%, respectively. At similar exposure, they are best compared with HC-1119 at 80 mg group. As shown in Figure 4 (HC-1119, 80 mg group clinical PK), for HC-1119-MO, the CV for Day 84 $C_{max}$, $C_{trough}$, and $AUC_{last}$ are 13.21%, 14.52% and 14.04%, respectively. On all three measurements, HC-1119-MO demonstrated lower inter-patient variation. This is also the case for HC-1119-M1 and HC-1119-M2. For enzalutamide M1, the CV for steady state $C_{max}$, $C_{min}$, and $AUC_{tau}$ are 73.5%, 82.2% and 74.5%, respectively,while for HC-1119-M1, the CV for steady state $C_{max}$, $C_{trough}$, and $AUC_{last}$ are 50.56%, 63.64% and 55.04%, respectively. For enzalutamide M2, the CV for steady state $C_{max}$, $C_{min}$, and $AUC_{tau}$ are 29.7%, 30.9% and 30.7%, respectively, while for HC-1119-M2, the CV for steady state $C_{max}$, $C_{trough}$, and $AUC_{last}$ are 18.73%, 20.98% and 21.19%, respectively. The same conclusion can also be made for other HC-1119 dose groups by comparing the CV in Figure 3 (HC-1119 40 mg), Figure 5 (HC-1119 160 mg) and Figure 6 (HC-1119 200 mg) with those in Figure 7 (enzalutamide 160 mg). HC-1119 demonstrated reduced inter-patient variation or variability, and thus has better safety accordingly.

Figure 8 provides some PK parameters for 80 mg HC-1119 in comparison with those of 160 mg. of the non-deuterated drug, enzalutamide. The tables show that at steady state, the parameters for active plasma drug compounds (M0+M2) of 80 mg HC-1119 is close to those obtained at double the dosage, 160 mg, of enzalutamide in terms of $C_{max}$, $C_{trough}$, and $AUC_{0-24h}$.

Figures 9-12 show the plasma concentration versus time Curve at week 12 for patients dosed with 40 mg, 80 mg, 160 mg and 200 mg, respectively, of HC-1119.

Figure 13 shows the changes in $C_{24h}$ versus time for the parent drug (HC-1119-MO) during the treatment period of the trial (84 days) for patients dosed with 40 mg, 80 mg, 160 mg and 200 mg of HC-1119. It depicts the M0 concentration values as the mean $\pm$SD.

Figures 14 and 15 show the changes in $C_{24h}$ versus time for the first and second metabolites, M1 and M2, respectively, during the treatment period of the trial (84 days) for patients dosed with 40 mg, 80 mg, 160 mg and 200 mg of HC-1119. They depicts the M0 concentration values as the mean $\pm$SD.

Figures 16A and 16B show the changes in $C_{24h}$ versus time for the combination of the parent drug and its active, second metabolite (HC-1119 M0+M2) at steady state during treatment for patients dosed with either 80 mg HC-1119 (Figure 16A) or 160 mg enzalutamide (Figure 16B). As can be observed, the $C_{24h}$ for the combination of the active drugs (HC-1119 M0+M2) of 80 mg HC-1119 is close to that of 160 mg enzalutamide at steady state.

**Example 12. HC-1119 has higher Safety Margin**

As discussed above, enzalutamide (M0) has two major metabolites, inactive M1 and active M2. Deuteration stabilizes M0 by blocking the generation of M1 and M2. Also, enzalutamide has the risk of inducing epilepsy due to the brain penetration of M0 and M2.

Shown below is the pathway by which M1 and M2 are generated for HC-1119 and enzalutamide, respectively.

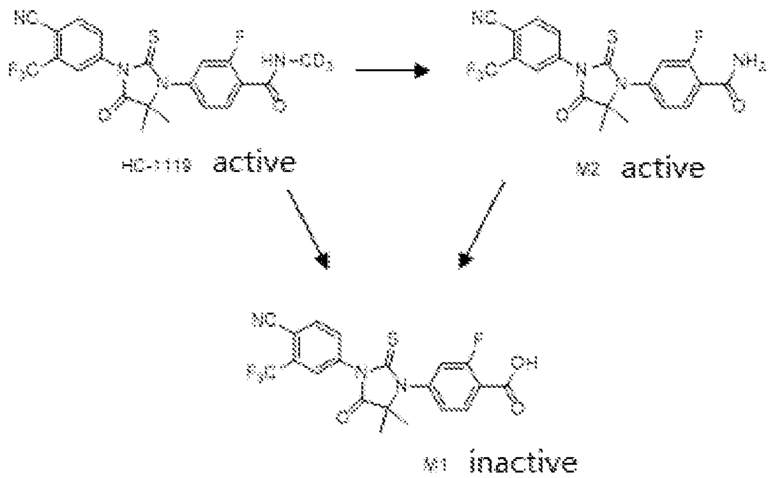


HC-1119

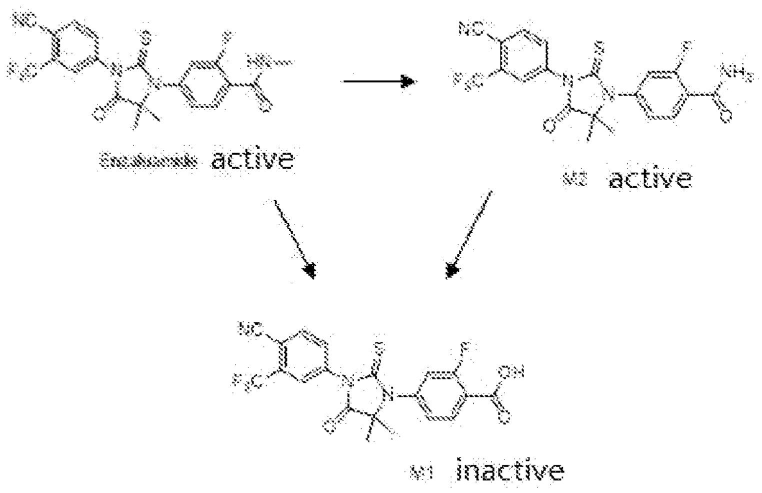


Enzalutamide

M1 is inactive; M0 and M2 are both active with similar activities (for AR binding, M0 Ki=51 nM, M2 Ki=57 nM; for LNCaP growth inhibition, M0 IC50=0.12 nM, M2 IC50=0.13 nM). In patients, at steady state of 160 mg/day of enzalutamide, the $C_{trough}$ ratio of MO:M2:M1 is 1:1:0.5 (obtained from FDA documents).

In patients at steady state of 80 mg/day of HC-1119, as provided in Figure 10, the $C_{trough}$ ratio of MO:M2:M1 is 1:0.18:0.1, showing that the deuteration stabilizes M0 by blocking the generation of M2 and M1. Patients, at steady state 80 mg/day HC-1119 had a PK profile similar to 160 mg/day enzalutamide in terms of $C_{max}$, $C_{trough}$, and AUC0-24h, showing the higher efficacy of HC-1119.

The following table shows that M2 is more readily to cross the blood-brain barrier (BBB) in Mice. Table 2 shows the brain penetration of parental drug (M0), M1, and M2 in the PD study in which study, mice (N=10, po, qd) were dosed were with 10 mg/kg of compounds (HC-1119 or enzalutamide) for 28 consecutive days and the parental drug and their major metabolites M1/M2 analyzed from samples collected at 24 h post last dosing.

**Table 2.Brain penetration of parental drug (M0), M1, and M2**

| | Brain (ng/g) | Plasma (ng/ml) | Brain/Plasma | Brain penetration |
|---|---|---|---|---|
| HC-1119-MO | 1393 | 4686 | 0.3 | $\frac{M2(\frac{brain}{plasma})}{M0(\frac{brain}{plasma})} = 4$ |
| HC-1119-M1 | Not detected | 161 | Not calculated | |
| HC-1119-M2 | 18 | 15 | 1.2 | |
| Enzalutamide-M0 | 647 | 2098 | 0.31 | $\frac{M2(\frac{brain}{plasma})}{M0(\frac{brain}{plasma})} = 3$ |
| Enzalutamide-M1 | Not detected | 199 | Not calculated | |
| Enzalutamide-M2 | 48 | 51 | 0.94 | |

As shown above, M2 more readily crosses the BBB. Seizures occurred in approximately 1% of patients treated with enzalutamide in clinical trials. It is believed that the brain exposure of (M0+M2) is the cause of seizure inductions. The fact that HC-1119 had a great reduction in M2 both in animals and patients makes HC-1119 have less brain exposure to (M0+M2) than enzalutamide at the same plasma exposure levels. Thus, HC-1119 has a lower potential for inducing epilepsy in patients.

## Claims

1. Compound HC-1119, or a crystal form, pharmaceutically acceptable salt, hydrate or solvate thereof, for use in treating prostate cancer in a subject in need thereof, **characterized in that** said compound is administered to said subject in a therapeutically effective amount at a dosage of 80 mg/day, wherein the prostate cancer is castration resistant prostate cancer (CRPC) or metastatic castration resistant prostate cancer (mCRPC).

2. The compound for use of claim 1, the subject having a prostate specific antigen (PSA) that is about 5 ng/ml or higher ; wherein the plasma concentration of PSA is reduced by at least 50%.

3. The compound for use of claim 2, wherein in the subject the concentration of PSA is reduced by at least 60%, or at least 70%.

4. The compound for use of claim 2, wherein in the subject the concentration of PSA is reduced by at least 80%, or at least 90%.

5. The compound for use of any one of claims 1-4, wherein the HC-1119 is administered twice a day or,
wherein the HC-1119 is administered once a day.

6. The compound for use of any one of claims 1-5, wherein the HC-1119 is administered over a period of at least five (5) weeks, or at least six (6) weeks, or at least eight (8) weeks, or at least ten (10) weeks, or at least twelve (12) weeks.

7. The compound for use of any one of claims 1-6, wherein the HC-1119 is administered in an oral, parenteral, subcutaneous, intradermal, intramuscular, intravenous, intraarticular, intranasal, intramedullary, intraperitoneal, transmucosal, transdermal, rectal, topical, dermal, buccal, sublingual or intraocular manner, preferably wherein the HC-1119 is administered orally.

8. The compound for use of claim 1 or 2, wherein the subject has been previously diagnosed with prostate cancer,

   preferably wherein the subject currently has never been treated for said cancer or,
   preferably wherein the subject was previously diagnosed with prostate cancer and has undergone previous treatment for prostate cancer.

9. A compound HC-1119 for use in treating prostate cancer in a subject in need thereof, wherein upon administration of HC-1119 to the subject plasma concentrations of a combination of HC-1119 and its second metabolic product (HC-1119-M2) are produced that are at least 50% more than the plasma concentrations obtained with a combination of enzalutamide and its second metabolic product (M2) with an identical dosage of enzalutamide, wherein said

compound is administered to said subject in a therapeutically effective amount at a dosage of 80 mg/day; wherein the prostate cancer is castration resistant prostate cancer (CRPC) or metastatic castration resistant prostate cancer (mCRPC).

**10.** A compound HC-1119 for use in treating prostate cancer in a subject in need thereof, wherein upon administration of HC-1119 to the subject plasma concentrations of a combination of HC-1119 and its second metabolic product (HC-1119-M2) of at least about 10 μg/mL at steady state are produced, wherein the dosage of HC-1119 is 80 mg/day; wherein the prostate cancer is castration resistant prostate cancer (CRPC) or metastatic castration resistant prostate cancer (mCRPC).

**11.** Compound HC-1119 for use in treating prostate cancer in a subject in need thereof, wherein the inter-patient variability in the plasma concentrations or in the AUC is reduced compared to enzalutamide, **characterized in that** said compound HC- 1119 is administered to a subject in need thereof in a therapeutically effective amount at a dosage of 80 mg/day;
wherein the prostate cancer is CRPC or mCRPC.

**12.** The compound for use of claim 11, wherein the inter-patient variability in $C_{max}$, $C_{24h}$, and/or $C_{trough}$ are reduced.

**13.** The compound for use of claim 11, wherein the subject currently has been previously diagnosed with prostate cancer, preferably wherein the subject has never been treated or has undergone previous treatment for prostate cancer.

**14.** The compound for use of claim 13, wherein the HC-1119 is administered over a period of at least five (5) weeks.

## Patentansprüche

**1.** Verbindung HC-1119 oder Kristallform, pharmazeutisch unbedenkliches Salz, Hydrat oder Solvat davon zur Verwendung beim Behandeln von Prostatakrebs bei einem Individuum, bei dem diesbezüglich Bedarf besteht, **dadurch gekennzeichnet, dass** die Verbindung dem Individuum in einer therapeutisch wirksamen Menge in einer Dosierung von 80 mg/Tag verabreicht wird, wobei es sich bei dem Prostatakrebs um kastrationsresistenten Prostatakrebs (CRPC) oder kastrationsresistenten metastasierenden Prostatakrebs (mCRPC) handelt.

**2.** Verbindung zur Verwendung nach Anspruch 1, wobei das Individuum ein prostataspezifisches Antigen (PSA) aufweist, das mit etwa 5 ng/ml oder mehr vorliegt; wobei die Plasmakonzentration von PSA um mindestens 50 % reduziert wird.

**3.** Verbindung zur Verwendung nach Anspruch 2, wobei die Konzentration von PSA in dem Individuum um mindestens 60 % oder mindestens 70 % reduziert wird.

**4.** Verbindung zur Verwendung nach Anspruch 2, wobei die Konzentration von PSA in dem Individuum um mindestens 80 % oder mindestens 90 % reduziert wird.

**5.** Verbindung zur Verwendung nach einem der Ansprüche 1-4, wobei HC-1119 zweimal am Tag verabreicht wird oder wobei HC-1119 einmal am Tag verabreicht wird.

**6.** Verbindung zur Verwendung nach einem der Ansprüche 1-5, wobei HC-1119 über einen Zeitraum von mindestens fünf (5) Wochen oder mindestens sechs (6) Wochen oder mindestens acht (8) Wochen oder mindestens zehn (10) Wochen oder mindestens zwölf (12) Wochen verabreicht wird.

**7.** Verbindung zur Verwendung nach einem der Ansprüche 1-6, wobei HC-1119 auf eine orale, parenterale, subkutane, intradermale, intramuskuläre, intravenöse, intraartikuläre, intranasale, intramedulläre, intraperitoneale, transmukosale, transdermale, rektale, topische, dermale, bukkale, sublinguale oder intraokulare Art und Weise verabreicht wird, vorzugsweise wobei HC-1119 oral verabreicht wird.

**8.** Verbindung zur Verwendung nach Anspruch 1 oder 2, wobei das Individuum zuvor mit Prostatakrebs diagnostiziert wurde,
vorzugsweise wobei dieser Krebs bei dem Individuum zum gegenwärtigen Zeitpunkt noch nie behandelt wurde oder vorzugsweise wobei das Individuum zuvor mit Prostatakrebs diagnostiziert wurde und eine vorherige Behandlung

von Prostatakrebs durchlaufen hat.

9. Verbindung HC-1119 zur Verwendung beim Behandeln von Prostatakrebs bei einem Individuum, bei dem diesbezüglich Bedarf besteht, wobei Plasmakonzentrationen einer Kombination von HC-1119 und seinem zweiten Stoffwechselprodukt (HC-1119-M2) nach Verabreichung von HC-1119 an das Individuum produziert werden, die mindestens 50 % höher sind als die mit einer Kombination von Enzalutamid und seinem zweiten Stoffwechselprodukt (M2) bei einer identischen Dosierung von Enzalutamid erhaltenen Plasmakonzentrationen, wobei die Verbindung dem Individuum in einer therapeutisch wirksamen Menge in einer Dosierung von 80 mg/Tag verabreicht wird;
wobei es sich bei dem Prostatakrebs um kastrationsresistenten Prostatakrebs (CRPC) oder kastrationsresistenten metastasierenden Prostatakrebs (mCRPC) handelt.

10. Verbindung HC-1119 zur Verwendung beim Behandeln von Prostatakrebs bei einem Individuum, bei dem diesbezüglich Bedarf besteht, wobei Plasmakonzentrationen einer Kombination von HC-1119 und seinem zweiten Stoffwechselprodukt (HC-1119-M2) von mindestens etwa 10 µg/ml im Steady-State nach Verabreichung von HC-1119 an das Individuum produziert werden, wobei die Dosierung von HC-1119 80 mg/Tag beträgt;
wobei es sich bei dem Prostatakrebs um kastrationsresistenten Prostatakrebs (CRPC) oder kastrationsresistenten metastasierenden Prostatakrebs (mCRPC) handelt.

11. Verbindung HC-1119 zur Verwendung beim Behandeln von Prostatakrebs bei einem Individuum, bei dem diesbezüglich Bedarf besteht, wobei die Variabilität zwischen Patienten bei den Plasmakonzentrationen oder bei der AUC im Vergleich zu Enzalutamid reduziert ist, **dadurch gekennzeichnet, dass** die Verbindung HC-1119 einem Individuum, bei dem diesbezüglich Bedarf besteht, in einer therapeutisch wirksamen Menge in einer Dosierung von 80 mg/Tag verabreicht wird;
wobei es sich bei dem Prostatakrebs um CRPC oder mCRPC handelt.

12. Verbindung zur Verwendung nach Anspruch 11, wobei die Variabilitätzwischen Patienten bei $C_{max}$, $C_{24h}$ und/oder $C_{min}$ reduziert ist.

13. Verbindung zur Verwendung nach Anspruch 11, wobei das Individuum zum gegenwärtigen Zeitpunkt zuvor mit Prostatakrebs diagnostiziert wurde,
vorzugsweise wobei der Prostatakrebs bei dem Individuum noch nie behandelt wurde oder das Individuum eine vorherige Behandlung durchlaufen hat.

14. Verbindung zur Verwendung nach Anspruch 13, wobei HC-1119 über einen Zeitraum von mindestens fünf (5) Wochen verabreicht wird.

## Revendications

1. Composé HC-1119, ou une forme cristalline, un sel pharmaceutiquement acceptable, un hydrate ou un solvate de celui-ci, destiné à être utilisé dans le traitement du cancer de la prostate chez un sujet en ayant besoin, **caractérisé en ce que** ledit composé est administré en une quantité thérapeutiquement efficace à une dose de 80 mg/jour, le cancer de la prostate étant un cancer de la prostate résistant à la castration (CPRC) ou un cancer de la prostate résistant à la castration métastatique (CPRCm).

2. Composé selon la revendication 1, le sujet présentant un taux d'antigène spécifique de la prostate (PSA) supérieur ou égal à environ 5 ng/ml ;
la concentration plasmatique de PSA étant réduite d'au moins 50 %.

3. Composé selon la revendication 2, la concentration de PSA chez le sujet étant réduite d'au moins 60 %, ou d'au moins 70 %.

4. Composé selon la revendication 2, la concentration de PSA chez le sujet étant réduite d'au moins 80 %, ou d'au moins 90 %.

5. Composé selon l'une quelconque des revendications 1 à 4, le HC-1119 étant administré deux fois par jour ou, le HC-1119 étant administré une fois par jour.

6. Composé selon l'une quelconque des revendications 1 à 5, le HC-1119 étant administré pendant une période d'au moins cinq (5) semaines, ou au moins six (6) semaines, ou au moins huit (8) semaines, ou au moins dix (10) semaines, ou au moins douze (12) semaines.

7. Composé selon l'une quelconque des revendications 1 à 6, le HC-1119 étant administré par voie orale, parentérale, sous-cutanée, intradermique, intramusculaire, intraveineuse, intraarticulaire, intranasale, intramédullaire, intrapéritonéale, transmuqueuse, transdermique, rectale, topique, dermique, buccale, sublinguale ou intraoculaire, de préférence, le HC-1119 étant administré par voie orale.

8. Composé selon la revendication 1 ou 2, le sujet ayant été précédemment diagnostiqué avec un cancer de la prostate,

   de préférence, le sujet n'ayant jamais été traité pour ledit cancer ou,
   de préférence, le sujet ayant été précédemment diagnostiqué avec un cancer de la prostate et ayant déjà reçu un traitement pour le cancer de la prostate.

9. Composé HC-1119 destiné à être utilisé dans le traitement du cancer de la prostate chez un sujet en ayant besoin, chez lequel, après l'administration de HC-1119 au sujet, les concentrations plasmatiques d'une combinaison de HC-1119 et de son deuxième métabolite (HC-1119-M2) sont supérieures d'au moins 50 % aux concentrations plasmatiques obtenues avec une combinaison d'enzalutamide et de son deuxième métabolite (M2) à une dose identique d'enzalutamide, ledit composé étant administré audit sujet à une dose thérapeutiquement efficace, à raison de 80 mg/jour ;
   le cancer de la prostate étant un cancer de la prostate résistant à la castration (CPRC) ou un cancer de la prostate résistant à la castration métastatique (CPRCm).

10. Composé HC-1119 destiné au traitement du cancer de la prostate chez un sujet en ayant besoin, chez lequel, après l'administration de HC-1119 au sujet, des concentrations plasmatiques d'une combinaison de HC-1119 et de son deuxième métabolite (HC-1119-M2) d'au moins environ 10 $\mu$g/ml à l'équilibre sont obtenues, la posologie de HC-1119 étant de 80 mg/jour ;
   le cancer de la prostate étant un cancer de la prostate résistant à la castration (CPRC) ou un cancer de la prostate résistant à la castration métastatique (CPRCm).

11. Composé HC-1119 destiné au traitement du cancer de la prostate chez un sujet en ayant besoin, la variabilité inter-patients des concentrations plasmatiques ou de l'ASC est réduite par rapport à l'enzalutamide, ledit composé HC-1119 étant administré à un sujet en ayant besoin en une quantité thérapeutiquement efficace selon une posologie de 80 mg/jour ;
   le cancer de la prostate étant un CPRC ou un CPRCm.

12. Composé selon la revendication 11, la variabilité inter-patients de $C_{max}$, de $C_{24h}$ et/ou de $C_{min}$ étant réduite.

13. Composé selon la revendication 11, le sujet ayant été précédemment diagnostiqué avec un cancer de la prostate, de préférence, le sujet n'ayant jamais été traité ou ayant reçu un traitement précédent pour le cancer de la prostate.

14. Composé selon la revendication 13, le HC-1119 étant administré pendant une période d'au moins cinq (5) semaines.

Fig. 1B

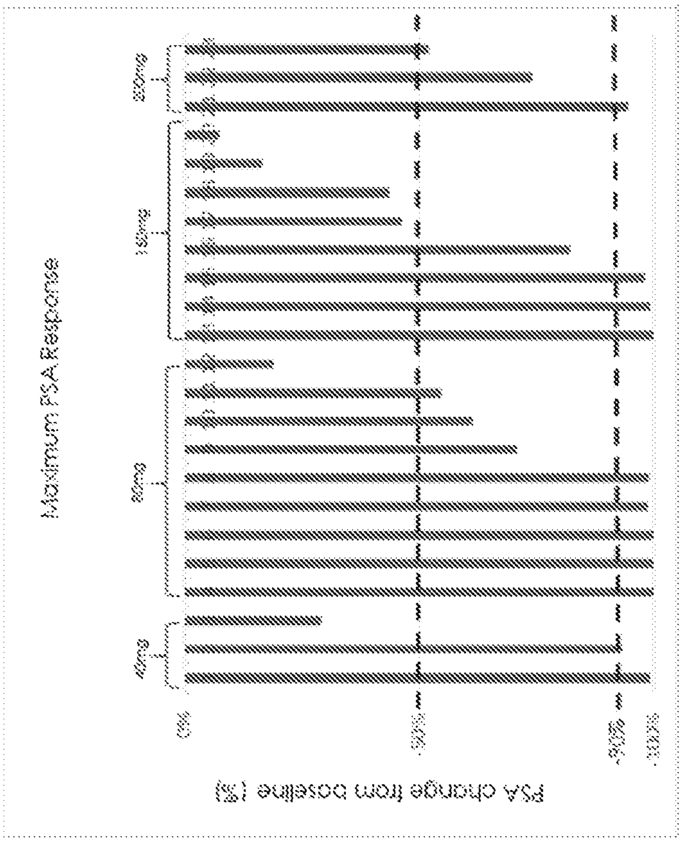
Maximum PSA Response
PSA change from baseline (%)
0%
-50%
-90%
-100%
40mg
80mg
160mg
200mg

Fig. 1A

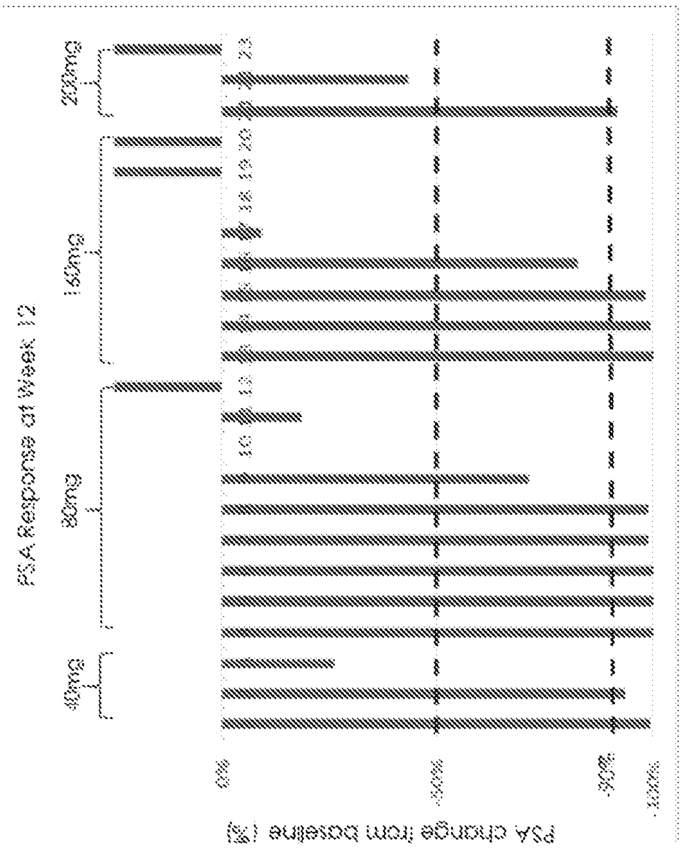
PSA Response at Week 12
PSA change from baseline (%)
0%
-50%
-90%
-100%
40mg
80mg
160mg
200mg

Fig. 2A

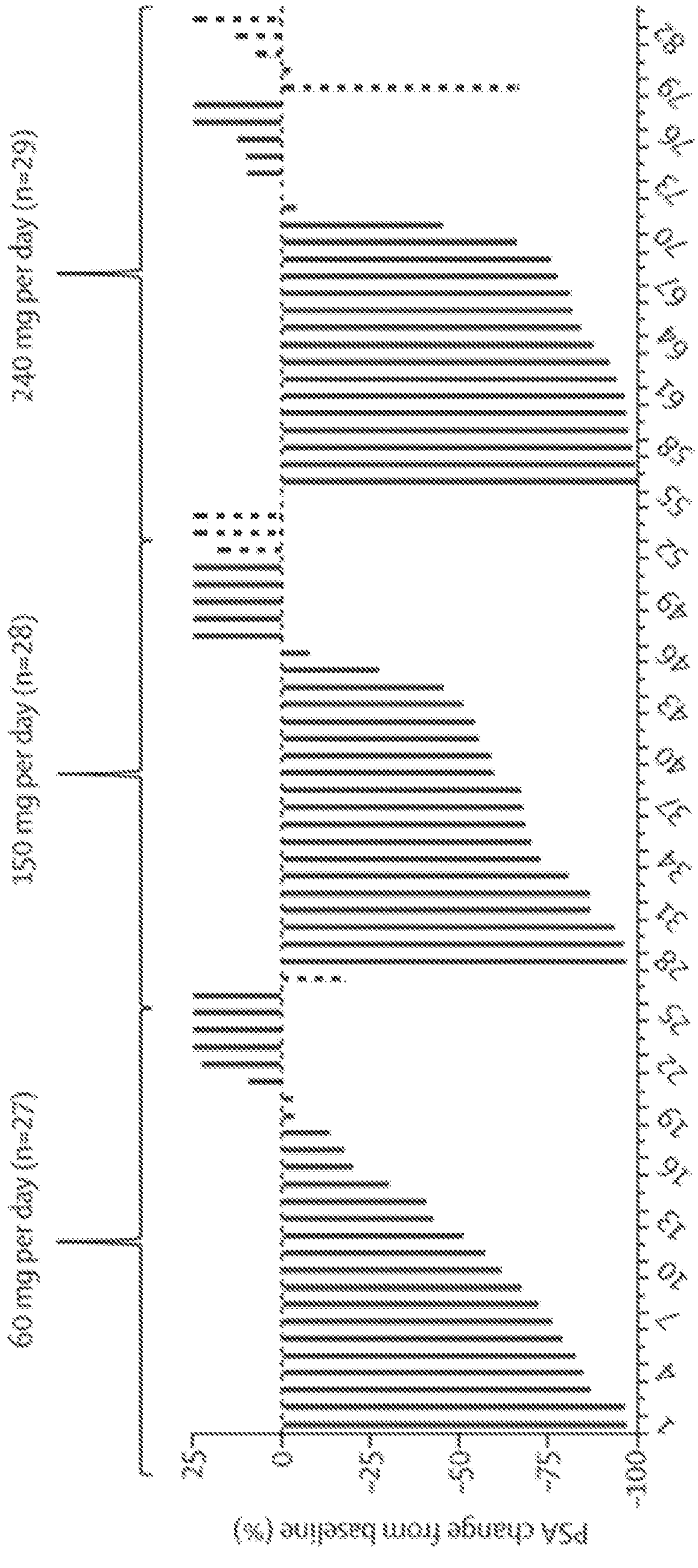
60 mg per day (n=27)
150 mg per day (n=28)
240 mg per day (n=29)
PSA change from baseline (%)
25
0
-25
-50
-75
-100
1
4
7
10
13
16
19
22
25
28
31
34
37
40
43
46
49
52
55
58
61
64
67
70
73
76
79
82

Fig. 2B

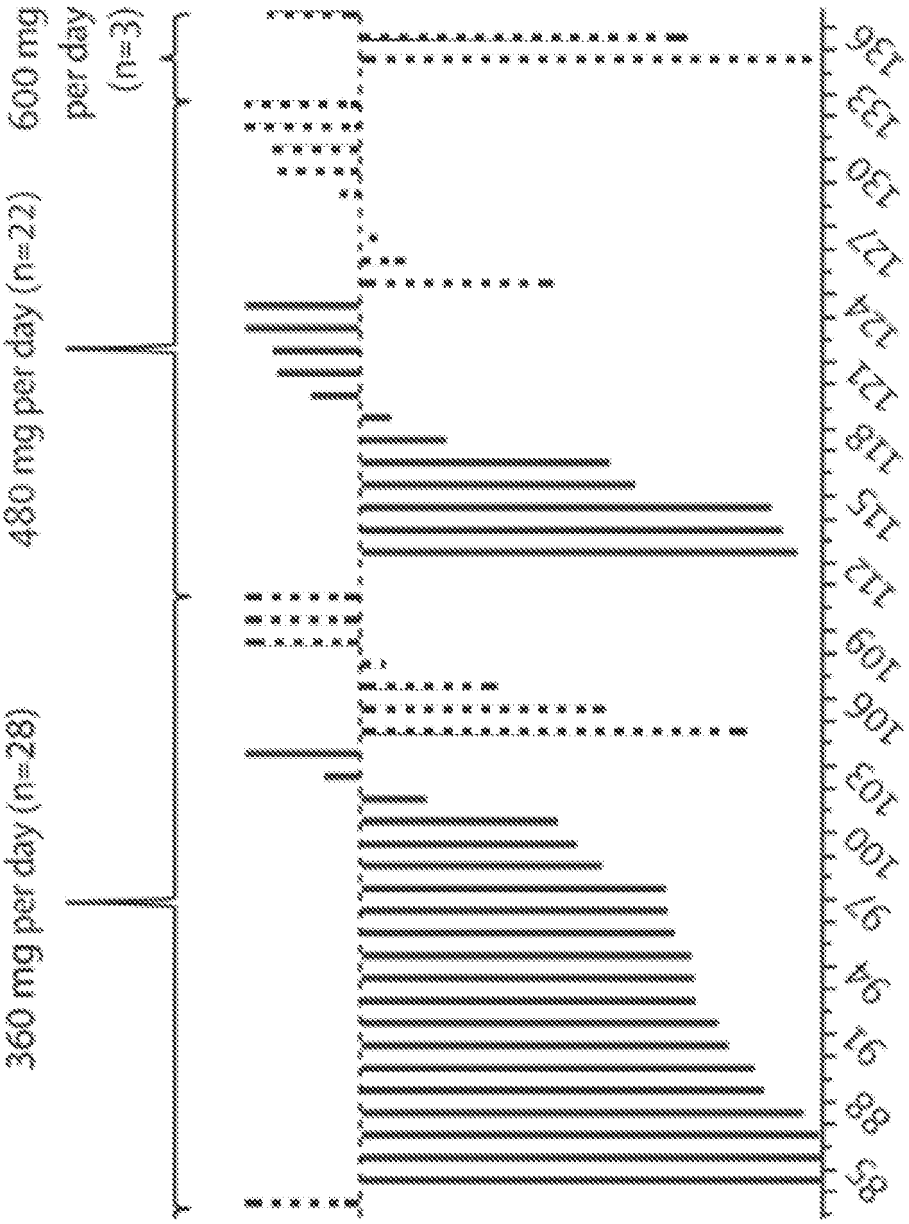
360 mg per day (n=28)
480 mg per day (n=22)
600 mg per day (n=3)
85
88
91
94
97
100
103
106
109
112
115
118
121
124
127
130
133
136

Fig. 3

HC-1119 Clinical PK Parameters (40 mg QD)

| **M0** | | | | | | | | | |
|---|---|---|---|---|---|---|---|---|---|
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 3 | 3 | 3 | 3 | 3 | 3 | 3 | 3 | 3 |
| Mean | 1122.33 | 1.25 | 12653.88 | 12766.67 | 11840.00 | 2000.58 | 10340.00 | 272551.25 | 1.26 |
| SD (CV%) | 263.91 (23.51) | 0.66 (52.92) | 2052.69 (16.22) | 2227.85 (17.45) | 2887.70 (24.39) | 13.35 (0.67) | 2791.20 (26.99) | 63895.72 (23.44) | 0.14 (11.41) |
| **M1** | | | | | | | | | |
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 3 | 3 | 3 | 3 | 3 | 3 | 3 | 3 | 3 |
| Mean | 30.80 | 24.00 | 546.87 | 1571.00 | 1178.00 | 1992.92 | 1102.67 | 32665.13 | 1.38 |
| SD (CV%) | 13.19 (42.81) | 0.00 (0.00) | 189.50 (34.65) | 1012.63 (64.46) | 523.58 (44.45) | 0.14 (0.01) | 592.71 (53.75) | 20682.01 (63.32) | 0.15 (11.10) |
| **M2** | | | | | | | | | |
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 3 | 3 | 3 | 3 | 3 | 3 | 3 | 3 | 3 |
| Mean | 17.30 | 24.00 | 207.36 | 2516.67 | 2466.67 | 2008.67 | 2040.00 | 55067.08 | 1.23 |
| SD (CV%) | 5.11 (29.54) | 0.00 (0.00) | 68.02 (32.80) | 110.15 (4.38) | 196.55 (7.97) | 12.70 (0.63) | 87.18 (4.27) | 2828.58 (5.14) | 0.04 (3.57) |

**Fig. 4**

HC-1119 Clinical PK Parameters (80 mg QD)

| **M0** | | | | | | | | | |
|---|---|---|---|---|---|---|---|---|---|
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 9 | 9 | 9 | 8 | 8 | 8 | 8 | 8 | 8 |
| Mean | 2841.11 | 1.00 | 29107.70 | 22912.50 | 20250.00 | 1993.50 | 18900.00 | 479745.31 | 1.22 |
| SD (CV%) | 523.87 (18.44) | 0.60 (59.95) | 8787.27 (30.19) | 3026.28 (13.21) | 3053.34 (15.08) | 1.56 (0.08) | 2744.34 (14.52) | 67360.15 (14.04) | 0.07 (5.46) |
| **M1** | | | | | | | | | |
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 9 | 9 | 9 | 8 | 8 | 8 | 8 | 8 | 8 |
| Mean | 68.62 | 16.44 | 1241.22 | 2931.25 | 2401.25 | 2002.19 | 2227.50 | 63584.69 | 1.38 |
| SD (CV%) | 21.00 (30.60) | 9.63 (58.57) | 336.85 (27.14) | 1482.03 (50.56) | 1385.40 (57.69) | 7.44 (0.37) | 1417.59 (63.64) | 34998.59 (55.04) | 0.17 (12.52) |
| **M2** | | | | | | | | | |
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 9 | 9 | 9 | 8 | 8 | 8 | 8 | 8 | 8 |
| Mean | 51.88 | 24.00 | 619.51 | 4060.00 | 3960.00 | 2003.00 | 3423.75 | 89350.16 | 1.19 |
| SD (CV%) | 36.60 (70.54) | 0.00 (0.00) | 367.06 (59.25) | 760.36 (18.73) | 841.12 (21.24) | 10.85 (0.54) | 718.31 (20.98) | 18934.57 (21.19) | 0.05 (3.95) |

**Fig. 5**

HC-1119 Clinical PK Parameters (160 mg QD)

| **M0** | | | | | | | | | |
|---|---|---|---|---|---|---|---|---|---|
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 9 | 9 | 9 | 8 | 8 | 8 | 8 | 8 | 8 |
| Mean | 4514.44 | 1.00 | 54569.71 | 43928.57 | 39071.43 | 1992.82 | 36785.71 | 927676.79 | 1.20 |
| SD (CV%) | 2196.28 (48.65) | 0.41 (41.46) | 10889.41 (19.96) | 4751.74 (10.82) | 4925.35 (12.61) | 0.19 (0.01) | 4386.13 (11.92) | 111747.97 (12.05) | 0.03 (2.29) |
| **M1** | | | | | | | | | |
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 9 | 9 | 9 | 8 | 8 | 8 | 8 | 8 | 8 |
| Mean | 130.81 | 18.67 | 2282.59 | 5428.57 | 4468.57 | 2002.57 | 3881.43 | 111370.36 | 1.44 |
| SD (CV%) | 75.52 (57.74) | 6.32 (33.88) | 1083.06 (47.45) | 2287.86 (42.14) | 1982.41 (44.36) | 7.87 (0.39) | 1819.37 (46.87) | 49914.95 (44.82) | 0.16 (11.35) |
| **M2** | | | | | | | | | |
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 9 | 9 | 9 | 8 | 8 | 8 | 8 | 8 | 8 |
| Mean | 90.78 | 24.00 | 1089.71 | 8090.00 | 7931.43 | 2000.18 | 7028.57 | 180753.57 | 1.15 |
| SD (CV%) | 35.62 (39.24) | 0.00 (0.00) | 418.16 (38.37) | 2154.21 (26.63) | 2055.48 (25.92) | 10.93 (0.55) | 1799.64 (25.60) | 46495.44 (25.72) | 0.02 (1.86) |

Fig. 6

HC-1119 Clinical PK Parameters (200 mg QD)

| **M0** | | | | | | | | | |
|---|---|---|---|---|---|---|---|---|---|
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 3 | 3 | 3 | 3 | 3 | 3 | 3 | 3 | 3 |
| Mean | 6786.67 | 0.92 | 67952.50 | 43366.67 | 38300.00 | 1992.83 | 35366.67 | 898762.50 | 1.23 |
| SD (CV%) | 869.85 (12.82) | 0.14 (15.75) | 14733.88 (21.68) | 2281.08 (5.26) | 2981.61 (7.78) | 0.14 (0.01) | 3370.95 (9.53) | 71468.63 (7.95) | 0.07 (6.00) |
| **M1** | | | | | | | | | |
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 3 | 3 | 3 | 3 | 3 | 3 | 3 | 3 | 3 |
| Mean | 110.33 | 20.00 | 2205.14 | 5636.67 | 4720.00 | 2000.33 | 4076.67 | 118839.58 | 1.37 |
| SD (CV%) | 11.37 (10.31) | 6.93 (34.64) | 163.09 (7.40) | 1527.49 (27.10) | 226.50 (4.80) | 6.35 (0.32) | 752.15 (18.45) | 23512.97 (19.79) | 0.13 (9.33) |
| **M2** | | | | | | | | | |
| **Parameter** | **Day1 Cmax (ng/mL)** | **Day1 Tmax (h)** | **Day1 AUC0-24h (h*ng/mL)** | **Day84 Cmax_ss (ng/mL)** | **Day84 C24h (ng/mL)** | **Day84 Tmax_ss (h)** | **Day84 Ctrough (ng/mL)** | **Day84_AUClast (h*ng/mL)** | **Day84 PTR** |
| N | 3 | 3 | 3 | 3 | 3 | 3 | 3 | 3 | 3 |
| Mean | 145.33 | 24.00 | 1758.95 | 8483.33 | 8480.00 | 2009.33 | 7453.33 | 190988.33 | 1.14 |
| SD (CV%) | 29.14 (20.05) | 0.00 (0.00) | 421.94 (23.99) | 1795.84 (21.17) | 1794.24 (21.16) | 11.55 (0.57) | 1612.03 (21.63) | 40757.35 (21.34) | 0.02 (1.35) |

**Fig. 7**

Enzalutamide Clinical PK Parameters (160 mg QD for 49 Days)

| **Enzalutamide** | | | | | | | |
|---|---|---|---|---|---|---|---|
| **Parameter** | **$AUC_{tau}$ (h*µg/mL)** | **$C_{0h}$ (µg/mL)** | **$C_{min}$ (µg/mL)** | **$C_{max}$ (µg/mL)** | **$t_{max}$ (h)** | **CL/F (L/h)** | **PTR** |
| N | 14 | 14 | 14 | 14 | 14 | 14 | 14 |
| Mean | 321.5 | 13.32 | 12.00 | 16.59 | NA | 0.520 | 1.266 |
| SD (CV%) | 85.39 (26.6) | 3.341 (25.1) | 3.512 (29.3) | 3.812 (23.0) | NA | 0.0942 (18.1) | 0.1271 (10.0) |
| Min-Max | 240-593 | 9.86-23.4 | 6.92-22.4 | 11.8-28.0 | 0.52-3.02 | 0.27-0.67 | 1.09-1.51 |
| Median | 295.6 | 12.70 | 11.45 | 15.55 | 1.02 | 0.541 | 1.240 |
| **M1** | | | | | | | |
| **Parameter** | **$AUC_{tau}$ (h*µg/mL)** | **$C_{0h}$ (µg/mL)** | **$C_{min}$ (µg/mL)** | **$C_{max}$ (µg/mL)** | **$t_{max}$ (h)** | **MPR (MWC)** | |
| N | 13 | 14 | 14 | 14 | 14 | 13 | |
| Mean | 193.3 | 7.739 | 6.315 | 8.867 | NA | 0.621 | |
| SD (CV%) | 144.01 (74.5) | 6.3111 (81.5) | 5.1909 (82.2) | 6.5201 (73.5) | NA | 0.4931 (79.4) | |
| Min-Max | 63.6-466 | 2.47-20.4 | 1.97-17.3 | 2.88-21.5 | 0.00-24.03 | 0.20-2.00 | |
| Median | 145.6 | 5.135 | 4.030 | 6.925 | 3.517 | 0.471 | |
| **M2** | | | | | | | |
| **Parameter** | **$AUC_{tau}$ (h*µg/mL)** | **$C_{0h}$ (µg/mL)** | **$C_{min}$ (µg/mL)** | **$C_{max}$ (µg/mL)** | **$t_{max}$ (h)** | **MPR (MWC)** | |
| N | 14 | 14 | 14 | 14 | 14 | 14 | |
| Mean | 278.3 | 11.97 | 10.57 | 12.68 | NA | 0.913 | |
| SD (CV%) | 85.47 (30.7) | 3.716 (31.0) | 3.271 (30.9) | 3.773 (29.7) | NA | 0.2812 (30.8) | |
| Min-Max | 182-442 | 7.80-18.8 | 6.99-16.5 | 8.65-19.6 | 0.00-24.03 | 0.56-1.66 | |
| Median | 263.2 | 11.15 | 10.23 | 12.10 | 4.02 | 0.886 | |

**Fig. 8**

(M0+M2) of 80 mg HC-1119 vs 160 mg Enzalutamide

| | HC-1119 (M0+M2) | | | Enzalutamide (M0+M2)<br>*Study number: 9785-CL-0007 |
|---|---|---|---|---|
| Dose (QD) | 40 mg (N=3) | **80 mg (N=8)** | 160 mg (N=8) | **160 mg** |
| $C_{max}$ (μg/ml) | 15.29±2.17 | **26.97±3.50** | 52.02±6.66 | **29.27±7.58** |
| $C_{trough}$ (μg/ml) | 12.38±2.71 | **22.32±3.22** | 43.81±5.90 | **22.57±6.78** |
| $AUC_{0-24h}$ (h*μg/ml) | 327.62±61.55 | **569.0±79.36** | 1108.43±150.88 | **599.8±171.13** |

**Fig. 9**

40 mg HC-1119: Plasma Concentration-time Curve at week 12

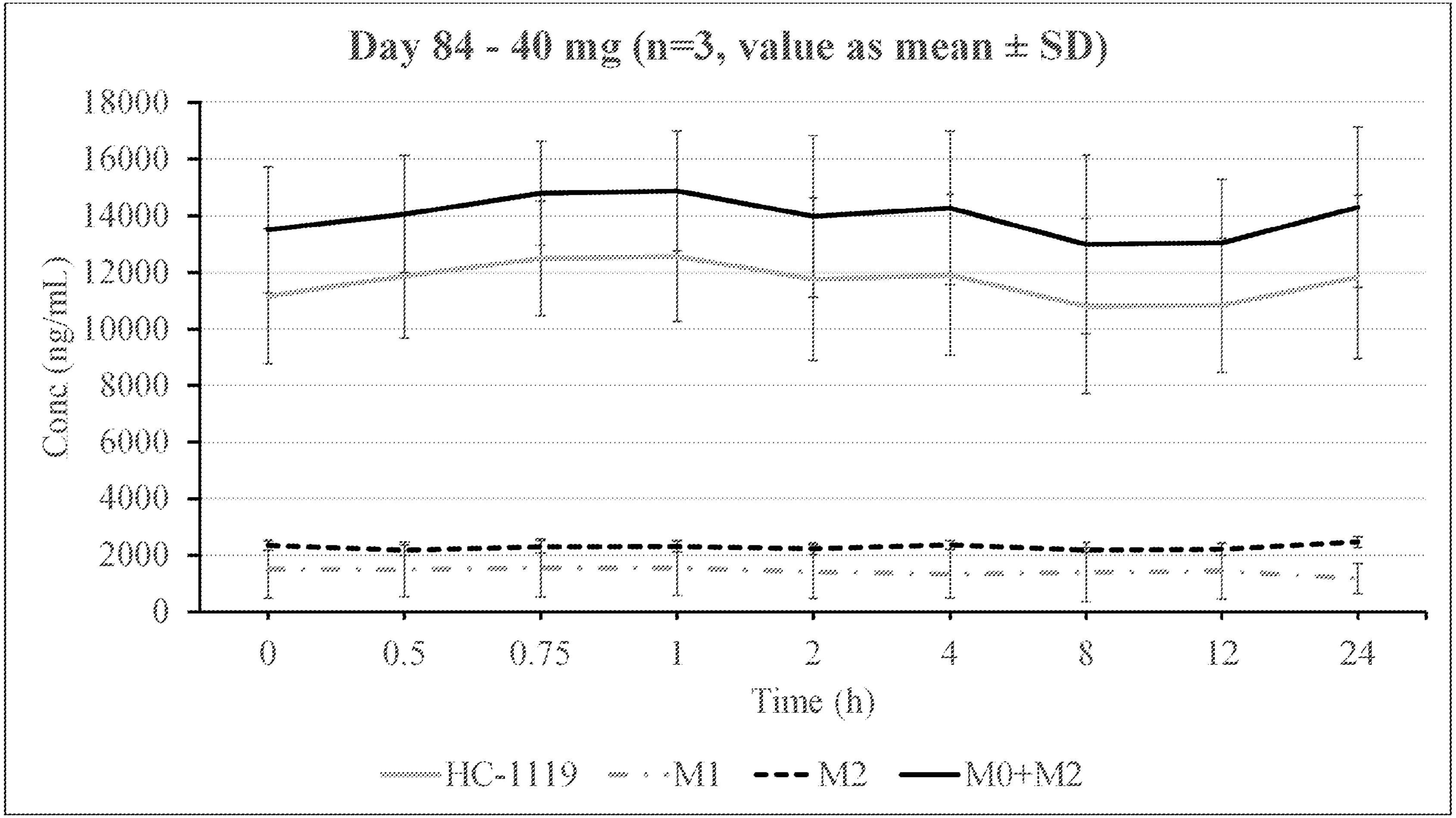

**Fig. 10**

80 mg HC-1119: Plasma Concentration-time Curve at week 12

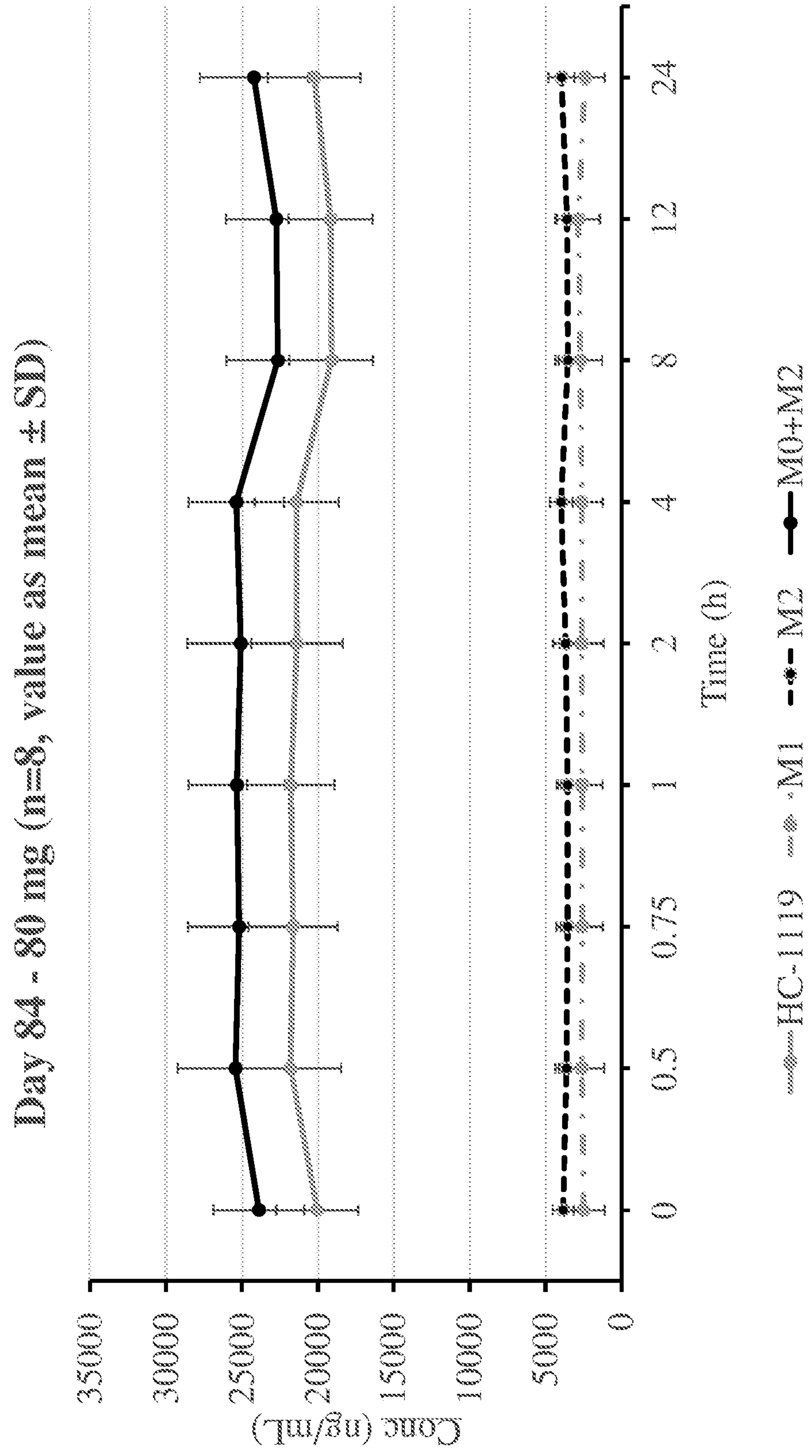

**Fig. 11**

160 mg HC-1119: Plasma Concentration-time Curve at week 12

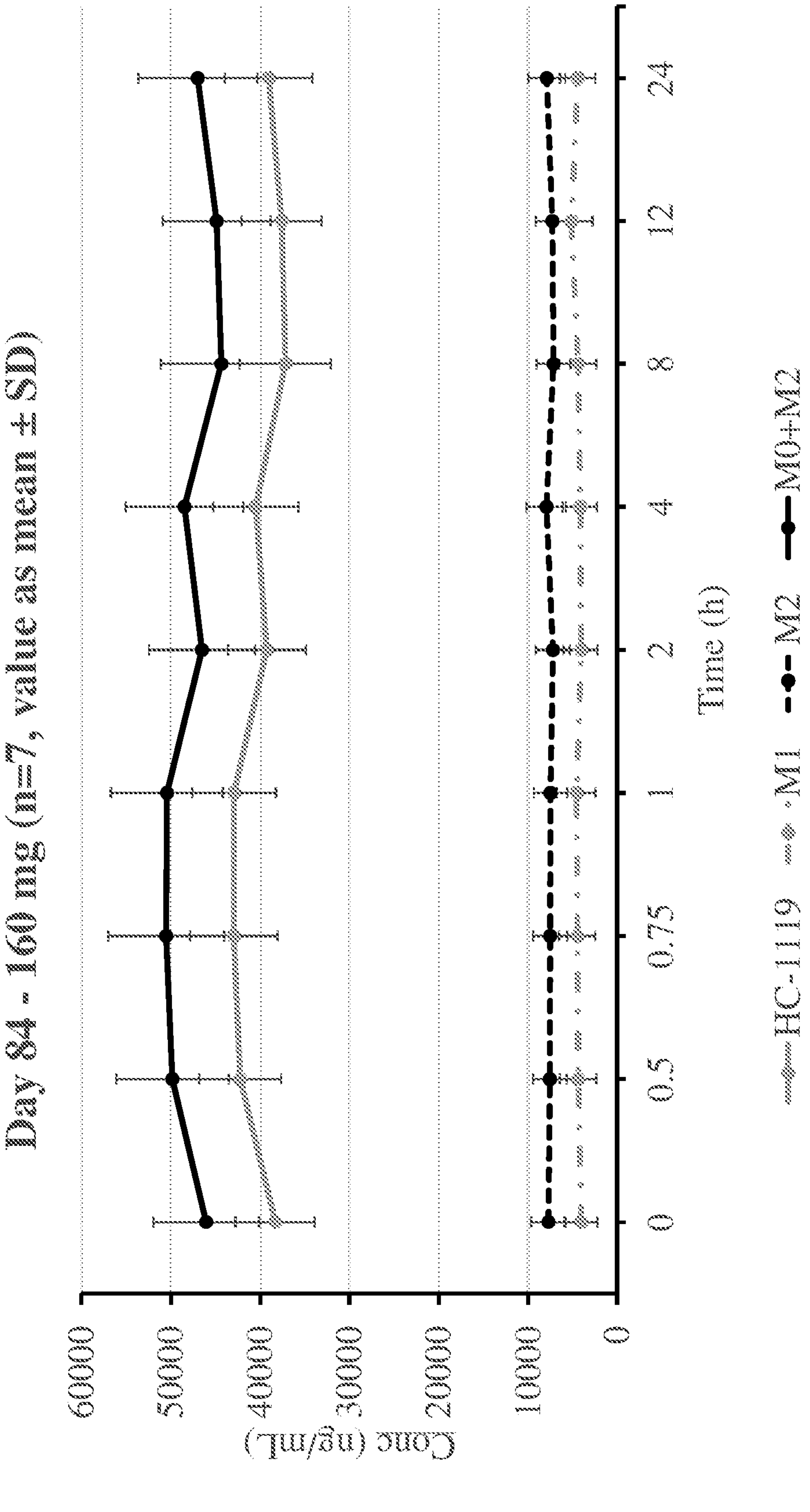

**Fig. 12**

200 mg HC-1119: Plasma Concentration-time Curve at week 12

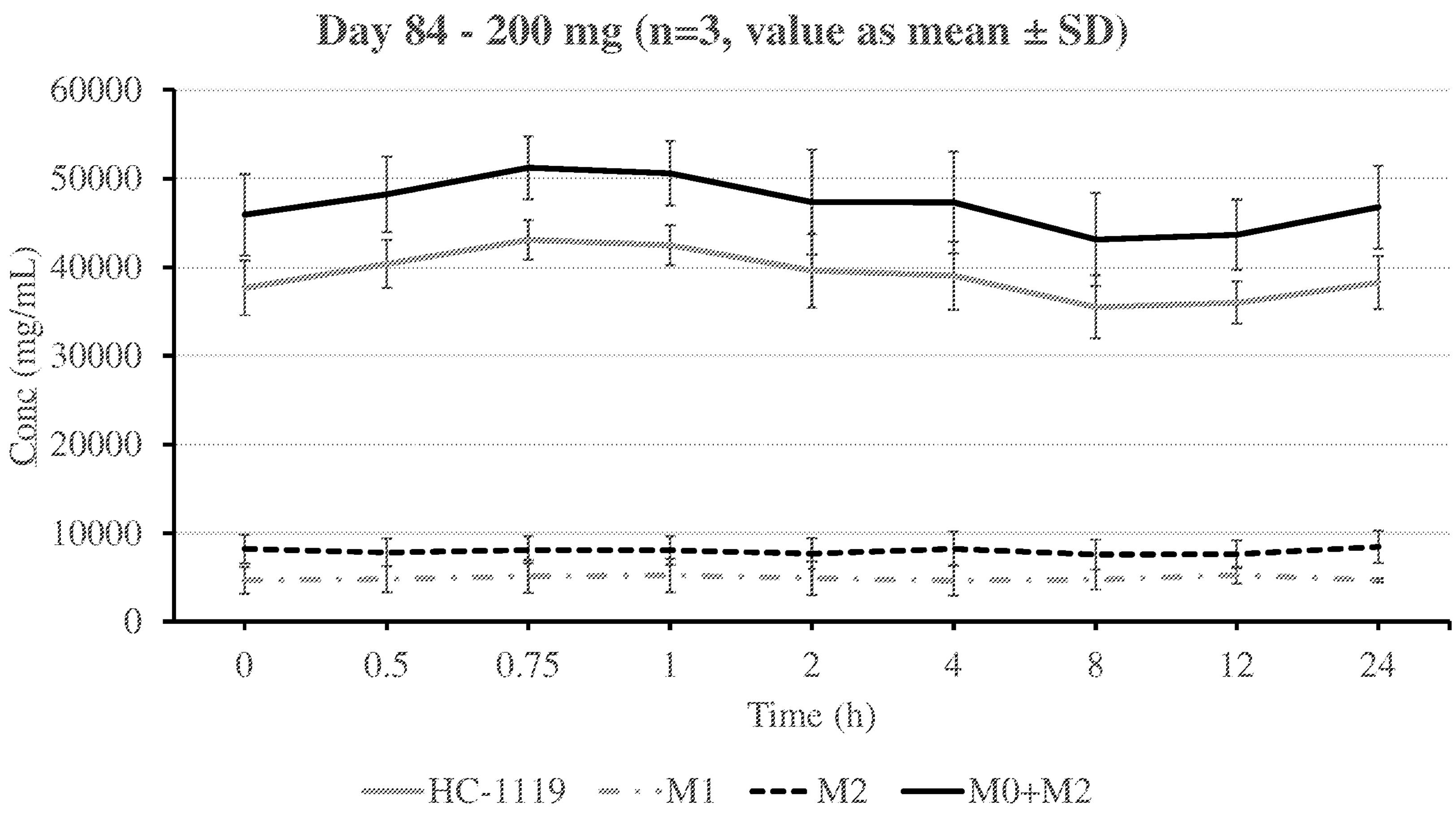

**Fig. 13**

M0 $C_{24h}$ Changes during Treatment (84 days)

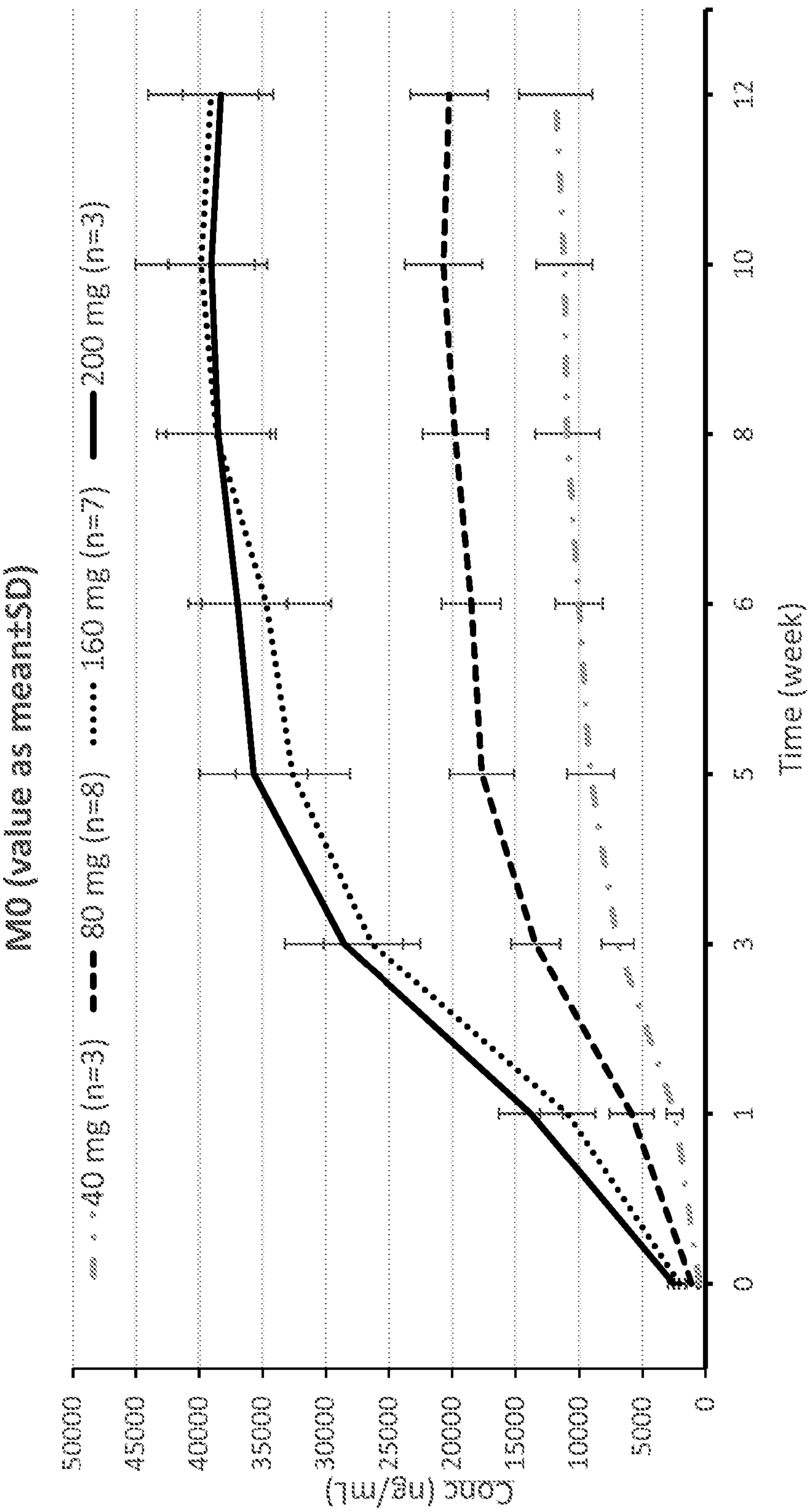

Fig. 14

M1 $C_{24h}$ Changes during Treatment (84 days)

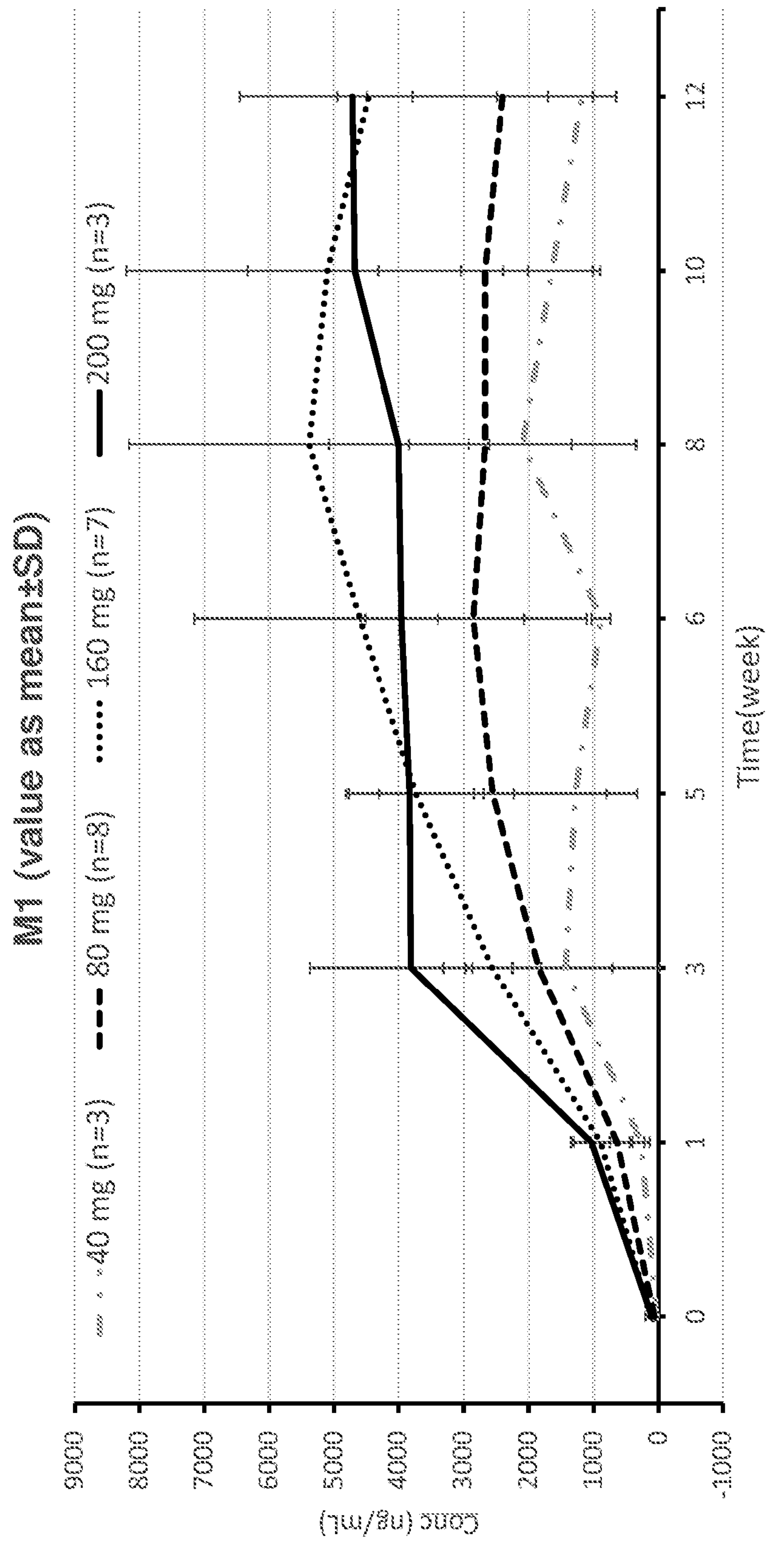

**Fig. 15**

M2 $C_{24h}$ Changes during Treatment (84 days)

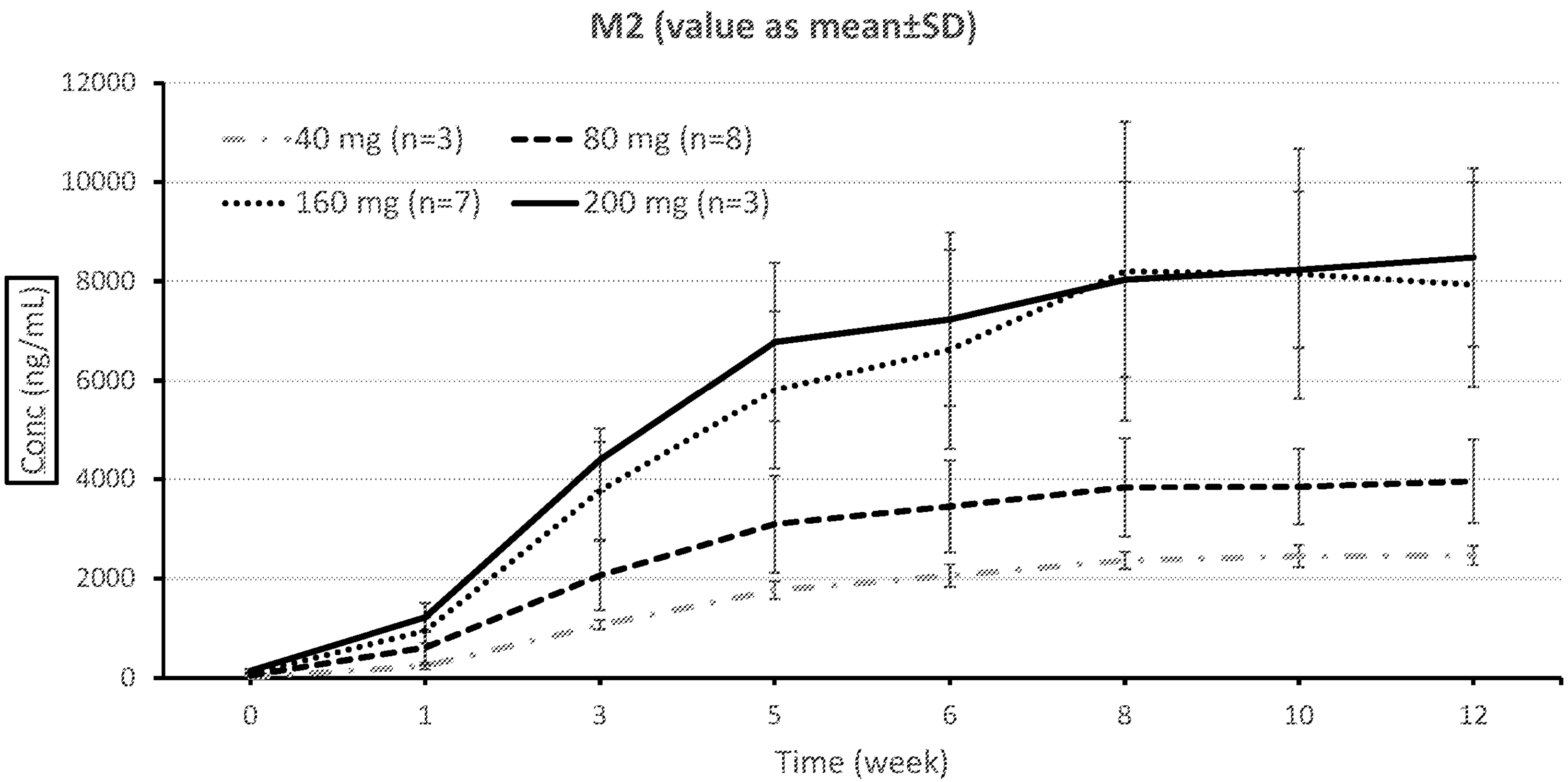

**Fig. 16A**

$C_{24h}$ (M0+M2) of 80 mg HC-1119 at Steady State

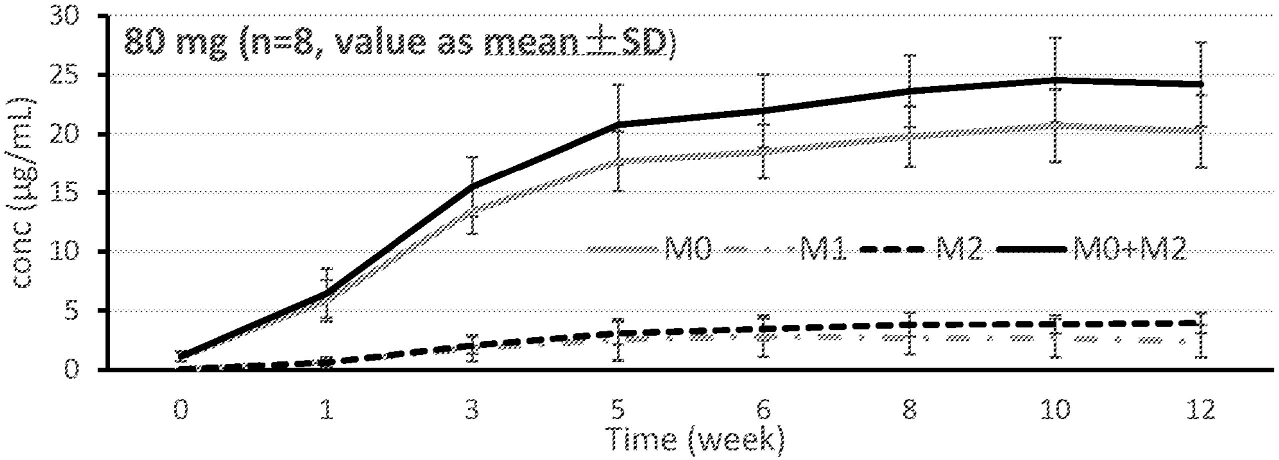


**Fig. 16B**

$C_{24h}$ (M0+M2) of 160 mg Enzalutamide at Steady State

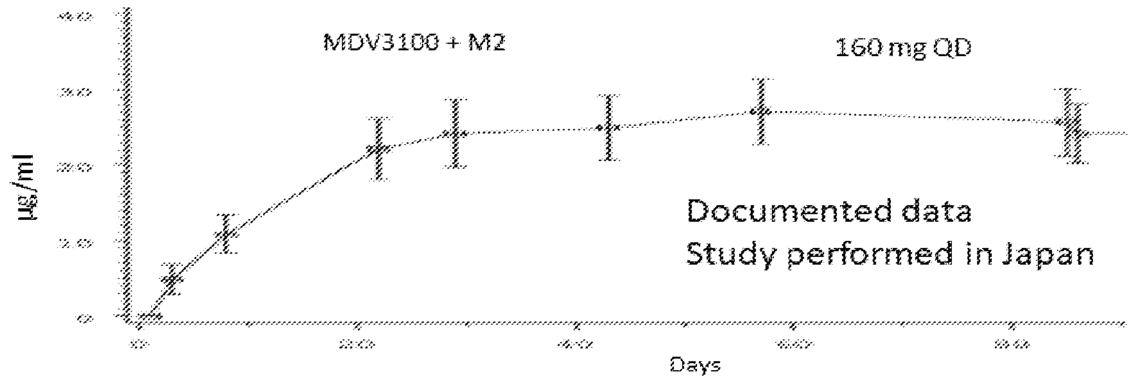

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **PANG, XUEHAI** ; **LINGLING PENG** ; **YUANWEI CHEN.** Effect of N-methyl deuteration on pharmacokinetics and pharmacodynamics of enzalutamide. *Journal of Labelled Compounds and Radiopharmaceuticals*, 2017, vol. 60 (9), 401-409